# EUROPEAN PATENT APPLICATION

(11) **EP 3 375 887 A1**
(43) Date of publication of application: **19.09.2018**
(21) Application number: 16864581.0
(22) Date of filing: 10.11.2016
(51) Int. Cl.: C12Q 1/68, C12N 15/113, A61K 48/00

(54) **METHODS FOR DETERMINING RESISTANCE TO ANTICANCER THERAPY AND COMPOSITION USED THEREFOR**

(30) Priority: 10.11.2015 KR 20150157539
(71) Applicant: SD Genomics Co., Ltd., Seoul 06336 (KR)
(72) Inventor: KIM, Jong-Won, Seoul 04392 (KR); PARK, Jong-Ho, Seoul 07535 (KR); WOO, Young-Min, Seoul 05643 (KR); PARK, Inho, Seoul 06336 (KR); PARK, Kyung Sun, Seoul 06336 (KR)
(74) Representative: Icosa
(86) International application number: PCT/KR2016/012948
(87) International publication number: WO 2017/082655

(57) **Abstract**

The present invention relates to a pharmaceutical composition for the prevention or treatment of malignant tumors comprising an agent capable of inhibiting the expression of HMGCLL1 IS3, a pharmaceutical composition for the prevention or treatment of malignant tumors showing resistance to tyrosine kinase inhibitors, a method for determining drug resistance in a subject having a malignant tumor using the expression level of HMGCLL1 IS3, a composition and a kit for diagnosing drug resistance used for the method, a method for determining the probability of developing a malignant tumor using the expression level of HMGCLL1 IS3, a method for screening malignant tumor therapeutic agents using the expression level of HMGCLL1 IS3, a method for predicting prognosis of malignant tumors using the expression level of HMGCLL1 IS3, a composition and a kit for predicting the prognosis of malignant tumors including an agent capable of measuring the expression level of HMGCLL1 IS3, a method for determining drug resistance in a subject having a malignant tumor using SNP, a composition and a kit for diagnosing drug resistance used for the method, a method for determining the probability of developing a malignant tumor using SNP, a method for predicting the prognosis of malignant tumors using SNP, and a composition and a kit for predicting the prognosis of malignant tumors used for the method. The method of the present invention can effectively predict or diagnose drug tolerance of a patient with chronic myeloid leukemia or a malignant tumor, and can further be used for efficient drug therapy and drug development research.

<110>    SD Genomics Co., Ltd.

<120>    Methods and compositions for determining resistance of cancer
         treatment

<130>    OPA16283-PCT

<150>    KR 10-2015-0157539
<151>    2015-11-10

<160>    178

<170>    KopatentIn 2.0

<210>    1
<211>    1113
<212>    DNA
<213>    Artificial Sequence

<220>
<223>    HMGCLL1 cDNA


<400>    1
atggggaatg tgccatccgc ggtgaagcac tgcctcagct accagcagct tctccgggag        60

catctctgga tcggggattc agtggcaggg gcgctcgacc ccgcgcagac ttctcttcta       120

acaaaccttc actgctttca gcccgatgtc tctggcttct cagtctcctt ggcaggcacg       180

gtggcttgta tccactggga aacatcccag ttatctggac tccctgagtt tgttaaaata       240

gtagaagttg ggcctaggga tggattgcag aatgaaaagg ttatagttcc tacagatata       300

aaaattgaat ttatcaatcg actttcccaa actggcttgt ctgtaataga agtgactagc       360

tttgtgtctt ccagatgggt accacagatg gctgatcaca ctgaagtaat gaaaggcatt       420

catcaatatc caggagttcg ctatcctgtc cttactccta atcttcaggg ttttcaccat       480

gctgttgctg ctggagctac tgagatatca gtttttggag ctgcatctga atcctttagc       540

aagaagaata ttaactgttc cattgaagaa agtatgggaa aatttgagga ggttgttaag       600

tctgcaagac acatgaatat tccagcacga gggtatgtgt cttgtgctct gggctgtcca       660

tatgaaggaa gtattacacc gcaaaaagtg acagaagtgt ctaagagatt gtacggcatg       720

ggttgttatg agatctctct aggagacaca attggagtgg gaactccagg aagtatgaaa       780

agaatgttgg aaagtgtgat gaaagaaatc ccaccaggtg ctcttgctgt tcactgtcat       840

gacacatacg gacaagcctt agcaaatatc cttacggccc ttcagatggg aattaatgtg       900

gtggactccg cagtatccgg attaggtggc tgcccttatg caaaaggtgc ttctgggaat       960

gtagccactg aggatttgat atatatgctt aatggcctgg ggctcaatac aggtgtgaat      1020

ctatacaaag tgatggaagc tggtgacttt atttgcaaag ctgtgaataa aaccacaaac      1080

tctaaagtag cacaagcctc cttcaatgct tga                                   1113

<210>    2
<211>    927
<212>    DNA
<213>    Artificial Sequence

<220>
<223>    HMGCLL1 IS3 cDNA


<400>    2
atggggaatg tgccatccgc ggtgaagcac tgcctcagct accagcagct tctccgggag      60

catctctgga tcggggattc agtggcaggg gcgctcgacc ccgcgcagga aacatcccag     120

ttatctggac tccctgagtt tgttaaaata gtagaagttg ggcctaggga tggattgcag     180

aatgaaaagg ttatagttcc tacagatata aaaattgaat ttatcaatcg actttcccaa     240

actggcttgt ctgtaataga agtgactagc tttgtgtctt ccagatgggt accacaggtt     300

gctgctggag ctactgagat atcagttttt ggagctgcat ctgaatcctt tagcaagaag     360

aatattaact gttccattga agaaagtatg ggaaaatttg aggaggttgt taagtctgca     420

agacacatga atattccagc acgagggtat gtgtcttgtg ctctgggctg tccatatgaa     480

ggaagtatta caccgcaaaa agtgacagaa gtgtctaaga gattgtacgg catgggttgt     540

tatgagatct ctctaggaga cacaattgga gtgggaactc caggaagtat gaaaagaatg     600

ttggaaagtg tgatgaaaga aatcccacca ggtgctcttg ctgttcactg tcatgacaca     660

tacggacaag ccttagcaaa tatccttacg gcccttcaga tgggaattaa tgtggtggac     720

tccgcagtat ccggattagg tggctgccct tatgcaaaag gtgcttctgg gaatgtagcc     780

actgaggatt tgatatatat gcttaatggc ctggggctca atacaggtgt gaatctatac     840

aaagtgatgg aagctggtga ctttatttgc aaagctgtga ataaaaccac aaactctaaa     900

gtagcacaag cctccttcaa tgcttga                                         927


<210>    3
<211>    308
<212>    PRT
<213>    Homo sapiens

<400>    3
Met Gly Asn Val Pro Ser Ala Val Lys His Cys Leu Ser Tyr Gln Gln
1               5                   10                  15

Leu Leu Arg Glu His Leu Trp Ile Gly Asp Ser Val Ala Gly Ala Leu
            20                  25                  30

Asp Pro Ala Gln Glu Thr Ser Gln Leu Ser Gly Leu Pro Glu Phe Val
        35                  40                  45

Lys Ile Val Glu Val Gly Pro Arg Asp Gly Leu Gln Asn Glu Lys Val
    50                  55                  60

Ile Val Pro Thr Asp Ile Lys Ile Glu Phe Ile Asn Arg Leu Ser Gln
65                  70                  75                  80

Thr Gly Leu Ser Val Ile Glu Val Thr Ser Phe Val Ser Ser Arg Trp
                85                  90                  95

Val Pro Gln Val Ala Ala Gly Ala Thr Glu Ile Ser Val Phe Gly Ala
            100                 105                 110

Ala Ser Glu Ser Phe Ser Lys Lys Asn Ile Asn Cys Ser Ile Glu Glu
        115                 120                 125

Ser Met Gly Lys Phe Glu Glu Val Val Lys Ser Ala Arg His Met Asn
    130                 135                 140

Ile Pro Ala Arg Gly Tyr Val Ser Cys Ala Leu Gly Cys Pro Tyr Glu
145                 150                 155                 160

Gly Ser Ile Thr Pro Gln Lys Val Thr Glu Val Ser Lys Arg Leu Tyr
                165                 170                 175

Gly Met Gly Cys Tyr Glu Ile Ser Leu Gly Asp Thr Ile Gly Val Gly
            180                 185                 190

Thr Pro Gly Ser Met Lys Arg Met Leu Glu Ser Val Met Lys Glu Ile
        195                 200                 205

Pro Pro Gly Ala Leu Ala Val His Cys His Asp Thr Tyr Gly Gln Ala
    210                 215                 220

Leu Ala Asn Ile Leu Thr Ala Leu Gln Met Gly Ile Asn Val Val Asp
225                 230                 235                 240

Ser Ala Val Ser Gly Leu Gly Gly Cys Pro Tyr Ala Lys Gly Ala Ser
                245                 250                 255

Gly Asn Val Ala Thr Glu Asp Leu Ile Tyr Met Leu Asn Gly Leu Gly
            260                 265                 270

Leu Asn Thr Gly Val Asn Leu Tyr Lys Val Met Glu Ala Gly Asp Phe
        275                 280                 285

Ile Cys Lys Ala Val Asn Lys Thr Thr Asn Ser Lys Val Ala Gln Ala
    290                 295                 300

Ser Phe Asn Ala
305


<210>    4
<211>    80
<212>    DNA
<213>    Artificial Sequence

<220>
<223>    cDNA fragment


<400>    4
agaagtgact agctttgtgt cttccagatg ggtaccacag gttgctgctg gagctactga        60

gatatcagtt tttggagctg                                                    80


<210>    5

<211>    19
<212>    RNA
<213>    Artificial Sequence

<220>
<223>    siRNA


<400>    5
agcagcaacc ugugguacc                                                    19


<210>    6
<211>    15
<212>    RNA
<213>    Artificial Sequence

<220>
<223>    siRNA


<400>    6
aaccuguggu accca                                                        15


<210>    7
<211>    15
<212>    RNA
<213>    Artificial Sequence

<220>
<223>    siRNA


<400>    7
gcagcaaccu guggu                                                        15


<210>    8
<211>    15
<212>    RNA
<213>    Artificial Sequence

<220>
<223>    siRNA


<400>    8
cuccagcagc aaccu                                                        15


<210>    9
<211>    51
<212>    DNA
<213>    Homo sapiens

<400>    9
tcatgaatat gaactgaatt ctcttmtttg acaagcggcc aaagccagct t                51


<210>    10
<211>    51
<212>    DNA

<213>    Homo sapiens

<400>    10
cagcttctgg acttctgctc ttctcyaaca atctattaca gtggttctca a                51


<210>    11
<211>    51
<212>    DNA
<213>    Homo sapiens

<400>    11
tagtctgtaa aaacatgata taaaasaaga cgtgaagtgt ttcagtcttt a                51


<210>    12
<211>    51
<212>    DNA
<213>    Homo sapiens

<400>    12
ttgctacatt catgtggaat acaccwttct gtaagcaaca caaaataaca t                51


<210>    13
<211>    51
<212>    DNA
<213>    Homo sapiens

<400>    13
atagtgaggt tggcaacttg gatacrccaa aaagaatctg caaagtgctt c                51


<210>    14
<211>    51
<212>    DNA
<213>    Homo sapiens

<400>    14
ctcctgaaac tggtgacccc aagccrttac tgcaggtaaa ggacatttca c                51


<210>    15
<211>    51
<212>    DNA
<213>    Homo sapiens

<400>    15
tgaatatgtt cccaatgatc tgggcyggac acttcaggta atacttcatg t                51


<210>    16
<211>    51
<212>    DNA
<213>    Homo sapiens

<400>    16
acgttatgta cagttctgag cgtgtrttaa aaatgtgtac acatgcatgt g                51


<210>    17
<211>    30
<212>    DNA

<213>    Artificial Sequence

<220>
<223>    primer


<400>    17
acgttggatg aaagccagct tctggacttc                                        30


<210>    18
<211>    30
<212>    DNA
<213>    Artificial Sequence

<220>
<223>    primer


<400>    18
acgttggatg agcaccaagg tgattctgtg                                        30


<210>    19
<211>    30
<212>    DNA
<213>    Artificial Sequence

<220>
<223>    primer


<400>    19
acgttggatg gccctcaaac taaacccaac                                        30


<210>    20
<211>    30
<212>    DNA
<213>    Artificial Sequence

<220>
<223>    primer


<400>    20
acgttggatg ggaacatgaa gtattacctg                                        30


<210>    21
<211>    30
<212>    DNA
<213>    Artificial Sequence

<220>
<223>    primer


<400>    21
acgttggatg tgagtccaaa cctattcctg                                        30


<210>    22

<211>    30
<212>    DNA
<213>    Artificial Sequence

<220>
<223>    primer


<400>    22
acgttggatg gatctcctga aactggtgac                                        30


<210>    23
<211>    30
<212>    DNA
<213>    Artificial Sequence

<220>
<223>    primer


<400>    23
acgttggatg cataggagct actttgctac                                        30


<210>    24
<211>    29
<212>    DNA
<213>    Artificial Sequence

<220>
<223>    primer


<400>    24
acgttggatg ggcatgttat tttgtgttg                                         29


<210>    25
<211>    30
<212>    DNA
<213>    Artificial Sequence

<220>
<223>    primer


<400>    25
acgttggatg aagtccagaa gctggctttg                                        30


<210>    26
<211>    30
<212>    DNA
<213>    Artificial Sequence

<220>
<223>    primer


<400>    26
acgttggatg agtgagaaaa ggcctctacc                                        30

<210>    27
<211>    30
<212>    DNA
<213>    Artificial Sequence

<220>
<223>    primer


<400>    27
acgttggatg taatgatgga cccaaagccc                                        30


<210>    28
<211>    31
<212>    DNA
<213>    Artificial Sequence

<220>
<223>    primer


<400>    28
acgttggatg cttaaaggaa gcactttgca g                                      31


<210>    29
<211>    30
<212>    DNA
<213>    Artificial Sequence

<220>
<223>    primer


<400>    29
acgttggatg ggtaaggtta ttaaagactg                                        30


<210>    30
<211>    31
<212>    DNA
<213>    Artificial Sequence

<220>
<223>    primer


<400>    30
acgttggatg gtatttgtgc ttgttgaggt c                                      31


<210>    31
<211>    30
<212>    DNA
<213>    Artificial Sequence

<220>
<223>    primer


<400>    31

acgttggatg ccacacactc actgaaacac                                        30

<210>    32
<211>    30
<212>    DNA
<213>    Artificial Sequence

<220>
<223>    primer

<400>    32
acgttggatg gtcccagacg ttatgtacag                                        30

<210>    33
<211>    51
<212>    DNA
<213>    Homo sapiens

<400>    33
gtggccctgt ggaagacgaa ctcatwaaat atggattatt atcattagca a                51

<210>    34
<211>    51
<212>    DNA
<213>    Homo sapiens

<400>    34
taagttctgg gatacatgtg cagaaygtgc agttttgtta cacaggtata c                51

<210>    35
<211>    51
<212>    DNA
<213>    Homo sapiens

<400>    35
aggccccggt gtgtgatgtt ccccty cctg tgtccatgtg ttctcattgt t               51

<210>    36
<211>    51
<212>    DNA
<213>    Homo sapiens

<400>    36
ctgtgtccat gtgttctcat tgttcwgctc ctacttatga gtgagaacat g                51

<210>    37
<211>    51
<212>    DNA
<213>    Homo sapiens

<400>    37
tttaaaagtt aagtcagtac aagaartttt gaatgtggat acatacaact t                51

<210>    38
<211>    51

<212>    DNA
<213>    Homo sapiens

<400>    38
aaagctaaga gattcctgaa tttggrctat tggagttatc agtgcttcta a    51


<210>    39
<211>    51
<212>    DNA
<213>    Homo sapiens

<400>    39
gttgtgtata tgtctttctt atgatrtcaa tgcgtttatc aaactctatg c    51


<210>    40
<211>    51
<212>    DNA
<213>    Homo sapiens

<400>    40
tgttcagctg caactggaag ttattntttc tgaagggctg tttctctctc t    51


<210>    41
<211>    51
<212>    DNA
<213>    Homo sapiens

<400>    41
aatatttccc aagtgcctgt catgtrcaga cactggttaa aattagattc a    51


<210>    42
<211>    51
<212>    DNA
<213>    Homo sapiens

<400>    42
gttaaaattt atcaaaactc agcacnacag actgtagata gtgccattct c    51


<210>    43
<211>    51
<212>    DNA
<213>    Homo sapiens

<400>    43
tcgcaggaca aagtgatctc ttgcasttct tatgtgtttt tcattatatt t    51


<210>    44
<211>    51
<212>    DNA
<213>    Homo sapiens

<400>    44
attttcaaac aaagaaacag gattcyaaga gcatgtagta aaggaaattg a    51


<210>    45
<211>    51

<212>    DNA
<213>    Homo sapiens

<400>    45
aggaacagct ttagctaaac tgaaasttta tcctgcctaa atttggattt t    51


<210>    46
<211>    51
<212>    DNA
<213>    Homo sapiens

<400>    46
ttggtgcaaa atttttcaaa aattamtaaa tttgtcaaaa ttaaccttaa a    51


<210>    47
<211>    51
<212>    DNA
<213>    Homo sapiens

<400>    47
aaaggccact cactacgtta tagccwcaaa aaaacaaaat gtatattttt a    51


<210>    48
<211>    51
<212>    DNA
<213>    Homo sapiens

<400>    48
atagcaacat ataacattca aagcakaaaa taccagtgaa atatatggtt t    51


<210>    49
<211>    51
<212>    DNA
<213>    Homo sapiens

<400>    49
ctagtgtgtt tagaaggctt agaatwaaga tattctataa gcttcttttg t    51


<210>    50
<211>    51
<212>    DNA
<213>    Homo sapiens

<400>    50
tgcctgtgtt tctgagatct tcacanctgt ctcatttcat gttcatatgc t    51


<210>    51
<211>    51
<212>    DNA
<213>    Homo sapiens

<400>    51
tccaaggcta atgtgatggt tctcarctat gttcccttaa ctgtatactt t    51


<210>    52
<211>    51

<212>    DNA
<213>    Homo sapiens

<400>    52
aactttacag aaaaaggttg tcaacycttt ctaaagaagt caagctgctt t                51


<210>    53
<211>    51
<212>    DNA
<213>    Homo sapiens

<400>    53
aaaagttata ttacatggac ctgacygtat ctaactaatc tcaggaattt c                51


<210>    54
<211>    51
<212>    DNA
<213>    Homo sapiens

<400>    54
aatgtttttg acttaagtga atacayagta gttctttcag caagagtctg g                51


<210>    55
<211>    51
<212>    DNA
<213>    Homo sapiens

<400>    55
tgtctctggt ctcagttatt gctgayagga attttactgt caagctaatt c                51


<210>    56
<211>    51
<212>    DNA
<213>    Homo sapiens

<400>    56
tttatttttc tgggtttcac tcttayagtt ttctcaaatc ttaattgcag g                51


<210>    57
<211>    51
<212>    DNA
<213>    Homo sapiens

<400>    57
tgcaggctaa gctgcacata atctgytgtt ccctttggcg acatgtaatc t                51


<210>    58
<211>    51
<212>    DNA
<213>    Homo sapiens

<400>    58
aagatctggt tttttttttt ttgctygcat tgtgaatttt ttaaagattc a                51


<210>    59
<211>    51

<212>    DNA
<213>    Homo sapiens

<400>    59
gtttatccat cttctgaatt ttattyttct catccacaaa gatgttatat c                51


<210>    60
<211>    51
<212>    DNA
<213>    Homo sapiens

<400>    60
tatgagcatc ttgaattatt taaacratcc tatcgaaaga atctgattgc t                51


<210>    61
<211>    51
<212>    DNA
<213>    Homo sapiens

<400>    61
ccttaccctg tggaatttat caattmttga acaaatttct accttaattt c                51


<210>    62
<211>    51
<212>    DNA
<213>    Homo sapiens

<400>    62
tgaacaaatt tctaccttaa tttctyaatt tgggtttcca caatactcat g                51


<210>    63
<211>    51
<212>    DNA
<213>    Homo sapiens

<400>    63
gactttgcat tggcaaagag gtgatrccag actccaagac atagttccaa a                51


<210>    64
<211>    51
<212>    DNA
<213>    Homo sapiens

<400>    64
ttttttctca gctaggtgat atgttytaaa agttataaaa tttctgtgtg t                51


<210>    65
<211>    51
<212>    DNA
<213>    Homo sapiens

<400>    65
ggacttagct aacccataga cccccntttt tcattctcat tacacacaca g                51


<210>    66
<211>    51

<212>    DNA
<213>    Homo sapiens

<400>    66
gatcaacccc aaagtgcctc atgtayatta caagcttgat gcattcacat a         51


<210>    67
<211>    51
<212>    DNA
<213>    Homo sapiens

<400>    67
gttaaacatg tctgaagtgg tcatcrtccc tatttctttc acataaacct g         51


<210>    68
<211>    51
<212>    DNA
<213>    Homo sapiens

<400>    68
aaccttctac ctttctttta ctatayatcc acaaaaaact ttatttcaat a         51


<210>    69
<211>    51
<212>    DNA
<213>    Homo sapiens

<400>    69
atttttcttc aaattatttt taaaanattt ttgattcata gttggttgaa t         51


<210>    70
<211>    51
<212>    DNA
<213>    Homo sapiens

<400>    70
tatgttctaa atcctgtagc cagaartaat agcacatatt tgagagatca g         51


<210>    71
<211>    51
<212>    DNA
<213>    Homo sapiens

<400>    71
atgaacaata tattttgaga gcctgwaatg catttattat tgtggataac a         51


<210>    72
<211>    51
<212>    DNA
<213>    Homo sapiens

<400>    72
attaaaaata ccttctgtat aaactrtgat ctctctacaa atatatgcaa a         51


<210>    73
<211>    51

<212>    DNA
<213>    Homo sapiens

<400>    73
ttctgtataa actgtgatct ctctayaaat atatgcaaag tttagaaaaa a                51


<210>    74
<211>    51
<212>    DNA
<213>    Homo sapiens

<400>    74
gttagaatat tttagggttt tctttkaaag aaaatggcat aacctccttc a                51


<210>    75
<211>    51
<212>    DNA
<213>    Homo sapiens

<400>    75
acctgagcct caagtaaatt aaatargttg cagaatacaa atagagtaaa c                51


<210>    76
<211>    51
<212>    DNA
<213>    Homo sapiens

<400>    76
cttaaaatga tgaatccttg gagtartttt taattaaaag gcctcagccc c                51


<210>    77
<211>    51
<212>    DNA
<213>    Homo sapiens

<400>    77
ctcagcccca cccagagcca cgtggnaaaa gtgttaagag tcccatactg g                51


<210>    78
<211>    51
<212>    DNA
<213>    Homo sapiens

<400>    78
tggggaaagt tttcaactta aaaagnaaaa aaaatattgt atgatgaagt t                51


<210>    79
<211>    51
<212>    DNA
<213>    Homo sapiens

<400>    79
agaaaaaaaa aatattgtat gatgahgttg ctaacatcta tggtaagaat a                51


<210>    80
<211>    51

<212>    DNA
<213>    Homo sapiens

<400>    80
acaaaaatta ctgaagaggc tatatvtgtg ggttaaactt tattatacat a         51


<210>    81
<211>    51
<212>    DNA
<213>    Homo sapiens

<400>    81
attactgaag aggctatatc tgtggrttaa actttattat acatatgtat g         51


<210>    82
<211>    51
<212>    DNA
<213>    Homo sapiens

<400>    82
ataaaaagag tatacatagg gtttgktaaa tgggacctgc atggcagact c         51


<210>    83
<211>    51
<212>    DNA
<213>    Homo sapiens

<400>    83
acttcaggta atacttcatg ttccanttta tgatgactta tattgtttgt t         51


<210>    84
<211>    51
<212>    DNA
<213>    Homo sapiens

<400>    84
ttcaacaaat taaaccaatg tagttycaga gacttttctc agagttctgt a         51


<210>    85
<211>    51
<212>    DNA
<213>    Homo sapiens

<400>    85
catttcattt catagctccc acattrtatt ttattttgta aacttcaaca g         51


<210>    86
<211>    51
<212>    DNA
<213>    Homo sapiens

<400>    86
tagctcccac attatatttt attttrtaaa cttcaacagc atgtctgctt t         51


<210>    87
<211>    51

<212>    DNA
<213>    Homo sapiens

<400>    87
tggttaacaa ggataaccat gtttartcca ggtgtccaca aatttttgaa c                51


<210>    88
<211>    51
<212>    DNA
<213>    Homo sapiens

<400>    88
atgctttcag agaatctggt tagatrtttc caaggtaaag ctctgagatt g                51


<210>    89
<211>    51
<212>    DNA
<213>    Homo sapiens

<400>    89
atattgaaaa agttaaataa gattaraaat aaatttatcg aatacttatt a                51


<210>    90
<211>    51
<212>    DNA
<213>    Homo sapiens

<400>    90
cagagtactt cttccaggta ttctgygttc taatttcata tgaatagtgt t                51


<210>    91
<211>    51
<212>    DNA
<213>    Homo sapiens

<400>    91
taaaagtaaa tttgtaaaca cttttmagga attcatagta aagttaatct c                51


<210>    92
<211>    51
<212>    DNA
<213>    Homo sapiens

<400>    92
cttctaaatg ttcatttttc tatttmatat gttgcacaac tctcttttca t                51


<210>    93
<211>    51
<212>    DNA
<213>    Homo sapiens

<400>    93
ttaatttcac accttaggga caccaytaac tttcttgaaa ttacttgtgt a                51


<210>    94
<211>    51

<212>    DNA
<213>    Homo sapiens

<400>    94
catagaggta gatcatccaa tggaarggat ttgatgctgt cataagaaat g                51


<210>    95
<211>    51
<212>    DNA
<213>    Homo sapiens

<400>    95
gagattctac attagacaag gagaasagga aagacacact caatgcaacg t                51


<210>    96
<211>    51
<212>    DNA
<213>    Homo sapiens

<400>    96
tttaagacca aattaagttt tccctktttt tgcataagaa gaaagtcaat a                51


<210>    97
<211>    51
<212>    DNA
<213>    Homo sapiens

<400>    97
cattttgtca ctgatttggc atggtrtctc ttcacaatgt gtcccagacg t                51


<210>    98
<211>    51
<212>    DNA
<213>    Homo sapiens

<400>    98
ttgtcactga tttggcatgg tatctyttca caatgtgtcc cagacgttat g                51


<210>    99
<211>    51
<212>    DNA
<213>    Homo sapiens

<400>    99
atagttttag tgtgctttta taaaaygtgt actgctatgg cctttaaagc t                51


<210>    100
<211>    51
<212>    DNA
<213>    Homo sapiens

<400>    100
tatataaatt gttgggagga ataagrtatt ttcaactaca caaatagctg c                51


<210>    101
<211>    51

<212>    DNA
<213>    Homo sapiens

<400>    101
cttatagtca catacttttt aacttyccaa tgagaattac tttaaagctt a                51


<210>    102
<211>    51
<212>    DNA
<213>    Homo sapiens

<400>    102
aatcgctatg cactttaact tgttamgatg tgtgttcatc atatctccat a                51


<210>    103
<211>    51
<212>    DNA
<213>    Homo sapiens

<400>    103
acaaaaatgc attataggtt tcacarccca taggcctggt tataatactt g                51


<210>    104
<211>    51
<212>    DNA
<213>    Homo sapiens

<400>    104
aaaaatgcat tataggtttc acaacmcata ggcctggtta taatacttga g                51


<210>    105
<211>    51
<212>    DNA
<213>    Homo sapiens

<400>    105
tctacttcct ctgggtgatc ccaaakcacc tccatagtat cttgcagaac a                51


<210>    106
<211>    51
<212>    DNA
<213>    Homo sapiens

<400>    106
tgcatgctag ttcctctgat ttcaaytgca tgacacattc cacaagctag a                51


<210>    107
<211>    51
<212>    DNA
<213>    Homo sapiens

<400>    107
tatcctgact tctacagcca tactcktgtg tacctaatgc tttgatcttt t                51


<210>    108
<211>    51

<212>    DNA
<213>    Homo sapiens

<400>    108
cactttttgt caactttatt ccttcytcca ggctctccag tgacttgttt t    51


<210>    109
<211>    51
<212>    DNA
<213>    Homo sapiens

<400>    109
acgttctccc ctcttgctaa tccccmatta acacatgggg gggaggtcag g    51


<210>    110
<211>    51
<212>    DNA
<213>    Homo sapiens

<400>    110
acagggagta aaagttctgg agtcaygttt tgagcacaga attgctccaa a    51


<210>    111
<211>    51
<212>    DNA
<213>    Homo sapiens

<400>    111
ttaactttgt aaatatcttt aaaacrataa gaggcctatc aattttagag a    51


<210>    112
<211>    51
<212>    DNA
<213>    Homo sapiens

<400>    112
gaaagtagtt ctcagtctca tagacktata ataagtagag ataagtttaa c    51


<210>    113
<211>    51
<212>    DNA
<213>    Homo sapiens

<400>    113
tagacttata ataagtagag ataagyttaa ctgttttaca gaatgggatc t    51


<210>    114
<211>    51
<212>    DNA
<213>    Homo sapiens

<400>    114
atgaacatgc aaaaagctgt gaaaayatgc caaaccaaaa ctggaatcct c    51


<210>    115
<211>    51

<212>    DNA
<213>    Homo sapiens

<400>    115
ttaccaggtg atcttttctc acttawttct atactattgt atccactatc c                51


<210>    116
<211>    51
<212>    DNA
<213>    Homo sapiens

<400>    116
tatctgactt taatctagct gtttaytgag cccctactat gtccaaagct c                51


<210>    117
<211>    51
<212>    DNA
<213>    Homo sapiens

<400>    117
cctataaaag tctgggagag tttgamagag cacatgggat acaagctgca g                51


<210>    118
<211>    51
<212>    DNA
<213>    Homo sapiens

<400>    118
agtgagcata gtaaatgttt acttaygcca ctaagttctc aggtaattca t                51


<210>    119
<211>    51
<212>    DNA
<213>    Homo sapiens

<400>    119
actctcaggt aattgtgaga atccakcttg tgacaagttc tgtggtagaa a                51


<210>    120
<211>    51
<212>    DNA
<213>    Homo sapiens

<400>    120
tcacattttg gtaattctca caatayttaa aactttttca ttattattat a                51


<210>    121
<211>    51
<212>    DNA
<213>    Homo sapiens

<400>    121
atgattaagt ttagtgagaa aggaaygttg aaagccccga taggtgataa a                51


<210>    122
<211>    51

<212>    DNA
<213>    Homo sapiens

<400>    122
ataaggaact gagagttcct attcawtttt tgacaagagt tgactcctat t                51


<210>    123
<211>    51
<212>    DNA
<213>    Homo sapiens

<400>    123
gctgcaccgg ccctgaacaa atgcarttac aaagctactc catgtacagg t                51


<210>    124
<211>    51
<212>    DNA
<213>    Homo sapiens

<400>    124
cagtaagtga gtctcagcca ttgtcnctgt ctccactcca cctatctgat a                51


<210>    125
<211>    51
<212>    DNA
<213>    Homo sapiens

<400>    125
gttctcctgc aatggtcttc aagtarcaca tggcgattgg aataccattt a                51


<210>    126
<211>    51
<212>    DNA
<213>    Homo sapiens

<400>    126
atgtacaaat ccaggccaga ctctgmcaat tgggagagct cacaattgct g                51


<210>    127
<211>    51
<212>    DNA
<213>    Homo sapiens

<400>    127
atattcacca tgttaatgtc ctacaytaac tggcaccaga agtatgtaaa t                51


<210>    128
<211>    51
<212>    DNA
<213>    Homo sapiens

<400>    128
ggaaattttc atctatcaaa aatatrttcc atataacaat tatactgtgc a                51


<210>    129
<211>    51

<212>    DNA
<213>    Homo sapiens

<400>    129
actgtagtag ttcttctaaa ataaaracta tatctgcgta caaatttaca t                51


<210>    130
<211>    51
<212>    DNA
<213>    Homo sapiens

<400>    130
aataagagaa aaagcgtatt atgaawtaac ctcagatgct cacttaaatt t                51


<210>    131
<211>    51
<212>    DNA
<213>    Homo sapiens

<400>    131
tgaattttga gatatttgta atatgygtta acttttaaaa ttttaaaatt t                51


<210>    132
<211>    51
<212>    DNA
<213>    Homo sapiens

<400>    132
atatgtgtta acttttaaaa ttttawaatt tcataagttc ttttttctca t                51


<210>    133
<211>    51
<212>    DNA
<213>    Homo sapiens

<400>    133
ggttggccat atatctccta gtgatwtgca gatctcaccc agagacagac a                51


<210>    134
<211>    51
<212>    DNA
<213>    Homo sapiens

<400>    134
ttcatatttc ctggttccag tgtttktgat gagggacctt gacagatgca a                51


<210>    135
<211>    51
<212>    DNA
<213>    Homo sapiens

<400>    135
taattactat tactattaat taataktaat agtaatagta attaatatta t                51


<210>    136
<211>    51

<212>    DNA
<213>    Homo sapiens

<400>    136
caccgggata tggaatcaga aaactnaaaa aagaattcta gagggcctta g    51


<210>    137
<211>    51
<212>    DNA
<213>    Homo sapiens

<400>    137
tagaaaagtc tgcaactcct ccctgbggtg aggagtagaa gttttggagt a    51


<210>    138
<211>    51
<212>    DNA
<213>    Homo sapiens

<400>    138
tacccccagc tggaacttcg ttatcrtctt cttactactg agattccaga a    51


<210>    139
<211>    51
<212>    DNA
<213>    Homo sapiens

<400>    139
atgttaaacc tatgggtatt taattmttta tgtggtatcc ttttacaaat t    51


<210>    140
<211>    51
<212>    DNA
<213>    Homo sapiens

<400>    140
tcacccagtt agtgagtgca aaagcygggc agggacccaa gtctatctga c    51


<210>    141
<211>    51
<212>    DNA
<213>    Homo sapiens

<400>    141
agagagaact caccttcagg ctgaavagtc ttgtggcata ttttatcatc t    51


<210>    142
<211>    51
<212>    DNA
<213>    Homo sapiens

<400>    142
tagagcatgc aatttcttca acacawgcca tcgattacta taaaaatgta a    51


<210>    143
<211>    51

<212>    DNA
<213>    Homo sapiens

<400>    143
ctacacgtct caagagtgac caaaayagga agcatatgac ccaaactctt c                51


<210>    144
<211>    51
<212>    DNA
<213>    Homo sapiens

<400>    144
gtctcaaagc tcccagtata attttytttc cactcaactg tgctgcctct c                51


<210>    145
<211>    51
<212>    DNA
<213>    Homo sapiens

<400>    145
ttccaattat ctattttaac tttacmctta ccttattagg tatatattat g                51


<210>    146
<211>    51
<212>    DNA
<213>    Homo sapiens

<400>    146
tagtagccca aatatagtca tccacrcccc gccccccaac cccccaccag a                51


<210>    147
<211>    51
<212>    DNA
<213>    Homo sapiens

<400>    147
ttcttgaaaa taagtactgc tagaamatac tcaaaactgg gctatccaga c                51


<210>    148
<211>    51
<212>    DNA
<213>    Homo sapiens

<400>    148
aaaaccagtc ctttgtgcca aaaagsctgg ggacctctga agtagaatat t                51


<210>    149
<211>    51
<212>    DNA
<213>    Homo sapiens

<400>    149
ttgtttctcc tatgtggtaa cagtarcaat gatttaattg tacagtggaa c                51


<210>    150
<211>    51

<212>    DNA
<213>    Homo sapiens

<400>    150
accctcacat acacctcgtc aaaatyacct ccttctttga ctcttttcca t                51


<210>    151
<211>    51
<212>    DNA
<213>    Homo sapiens

<400>    151
aagtaaattt atgaagatat aaagayaata aaaatgtcta gaactctctt a                51


<210>    152
<211>    51
<212>    DNA
<213>    Homo sapiens

<400>    152
acacacacac acactataaa cagcartgac aatgggatag agaaaattag t                51


<210>    153
<211>    51
<212>    DNA
<213>    Homo sapiens

<400>    153
aggcaggaca tgtaaataga ggacamgaaa cagttaattt agcagtagct t                51


<210>    154
<211>    51
<212>    DNA
<213>    Homo sapiens

<400>    154
tgctattctg ttatagtatc cctaastgac tacgacagta actggataca a                51


<210>    155
<211>    51
<212>    DNA
<213>    Homo sapiens

<400>    155
gggaaaacga aacagccttt gaaatwgtat attgcagtta aagtcagaag c                51


<210>    156
<211>    51
<212>    DNA
<213>    Homo sapiens

<400>    156
atgaattcaa gccaaccagt ttataytgtc acactctgtc cttgtaaaaa t                51


<210>    157
<211>    51

<212>    DNA
<213>    Homo sapiens

<400>    157
cctgatttac aagatgccta taattycaac agaacaataa ataaaatata t                51


<210>    158
<211>    51
<212>    DNA
<213>    Homo sapiens

<400>    158
atgcctcctg aatacagttg aagaaytgag gacctttctt tgcatcaatg t                51


<210>    159
<211>    51
<212>    DNA
<213>    Homo sapiens

<400>    159
tatgtcatgc attgggctgg ggctgrtata tgccaactta gagccagtaa t                51


<210>    160
<211>    51
<212>    DNA
<213>    Homo sapiens

<400>    160
tctctttcta tggctgtttg ctagamaaac attcttgaaa agatattctc a                51


<210>    161
<211>    51
<212>    DNA
<213>    Homo sapiens

<400>    161
aaaatttgga caagtaaaat tgaacwttct tttagctaac acatgctaag t                51


<210>    162
<211>    51
<212>    DNA
<213>    Homo sapiens

<400>    162
acaaaagatg ttttatccag agtgartgtt tcaaaaggct atgttttatg g                51


<210>    163
<211>    51
<212>    DNA
<213>    Homo sapiens

<400>    163
agacatattt gcttatgttt catgcrcttc atttcttgag aagtattttt t                51


<210>    164
<211>    51

<212>    DNA
<213>    Homo sapiens

<400>    164
agctttagga gagattaaaa acaagmtcta gaaatgataa aagccctctc t                51


<210>    165
<211>    51
<212>    DNA
<213>    Homo sapiens

<400>    165
aattccattt ttttaaaagt agcttvagca ataaatgcag ccaggtgttt a                51


<210>    166
<211>    51
<212>    DNA
<213>    Homo sapiens

<400>    166
ctatggtccc ttatacttct tgataygtga acagtgtttt atatttgttg g                51


<210>    167
<211>    51
<212>    DNA
<213>    Homo sapiens

<400>    167
aattttcagt tatttcatat tcttcraagt aggtgggata ttgctactcc c                51


<210>    168
<211>    51
<212>    DNA
<213>    Homo sapiens

<400>    168
aggacccagg actccactta ttatgyggtc ctcctatttc tcattttaac a                51


<210>    169
<211>    51
<212>    DNA
<213>    Homo sapiens

<400>    169
cacattgatc cactgtatca agactracca aagttctaac tatatgaaag g                51


<210>    170
<211>    51
<212>    DNA
<213>    Homo sapiens

<400>    170
tctaaagtct caaatagaca accccrtagt gatctatctc tgtagggcat a                51


<210>    171
<211>    51

<212>    DNA
<213>    Homo sapiens

<400>    171
tgactctatt ctgcaataaa aaatayattt tccatcccta gtatagtgaa a                51


<210>    172
<211>    51
<212>    DNA
<213>    Homo sapiens

<400>    172
aaatggaggt tacaatagct aaaaasagtt gtggtaaagc cacatatagt g                51


<210>    173
<211>    51
<212>    DNA
<213>    Homo sapiens

<400>    173
cacttactac tgagtggaca tttaaygtgt gtaggaccct gtgctagaat t                51


<210>    174
<211>    21
<212>    DNA
<213>    Artificial Sequence

<220>
<223>    primer


<400>    174
taccacaggt tgctgctgga g                                                 21


<210>    175
<211>    23
<212>    DNA
<213>    Artificial Sequence

<220>
<223>    primer


<400>    175
tgcagactta acaacctcct caa                                               23


<210>    176
<211>    19
<212>    RNA
<213>    Artificial Sequence

<220>
<223>    siRNA


<400>    176
gguaccacuc cuugcugcu                                                    19

<210> 177
<211> 19
<212> RNA
<213> Artificial Sequence

<220>
<223> siRNA

<400> 177
agcagcaagg agugguacc 19

<210> 178
<211> 19
<212> RNA
<213> Artificial Sequence

<220>
<223> siRNA

<400> 178
gguaccacag guugcugcu 19

## Description

### [Technical Field]

The present invention relates to a method for determining resistance to anticancer treatment and a composition used in the method, and more specifically, the present relates to a pharmaceutical composition for the prevention or treatment of malignant tumors containing an agent capable of inhibiting the expression of 3-hydroxymethyl-3-methylglutaryl-CoA lyase like 1 (hereinafter, HMGCLL1 IS3), a pharmaceutical composition for the prevention or treatment of malignant tumors showing resistance to tyrosine kinase inhibitors, a method for determining drug resistance in a subject having a malignant tumor using the expression level of HMGCLL1 IS3, a composition and a kit for diagnosing drug resistance used for the method, a method for determining the probability of developing a malignant tumor using the expression level of HMGCLL1 IS3, a method for screening malignant tumor therapeutic agents using the expression level of HMGCLL1 IS3, a method for predicting prognosis of malignant tumors using the expression level of HMGCLL1 IS3, a composition and a kit for predicting the prognosis of malignant tumors including an agent capable of measuring the expression level of HMGCLL1 IS3, a method for determining drug resistance in a subject having a malignant tumor using single nucleotide polymorphism (hereinafter, SNP), a composition and a kit for diagnosing drug resistance used for the method, a method for determining the probability of developing a malignant tumor using SNP, a method for predicting the prognosis of malignant tumors using SNP, and a composition and a kit for predicting the prognosis of malignant tumors used for the method.

### [Background Art]

Chronic myeloid leukemia (hereinafter, CML), which is a rare clonal myeloproliferative disorder, is a disease characterized by the proliferation of all of the myeloid cells due to abnormalities in hematopoietic stem cells. CML shows characteristics such as improved proliferative capacity, prolonged survival of hematopoietic stem cells (HSCs), and reduced apoptosis. CML is a very rare blood cancer which generally occurs in approximately 0.6 to 2 people per 100,000, and the incidence rate varies between Westerners and East Asians.

The BCR-ABL oncogene, which is known as the major cause of CML, is a gene structurally encoding activity of Bcr-Abl fusion tyrosine kinase (FTK) in the Philadelphia chromosome (Ph). Bcr-Abl FTK is known as an important molecular marker for CML, and imatinib has been developed as a therapeutic agent targeting BCR-ABL FTK. The Bcr-Abl recombinant gene induces proliferation of cancer cells by continuous activation of tyrosine kinase. Imatinib binds to an active site, which corresponds to a position where adenosine triphosphate (ATP) binds, and inhibits the activity of tyrosine kinase, thereby inducing a mechanism of action that inhibits proliferation of cancer cells. However, as time has progressed since the development of imatinib, there has been an increasing number of reports with respect to patients having resistance to imatinib.

The point mutation generated in the Bcr-Abl fusion gene has been identified as a cause of imatinib resistance. However, imatinib resistance has also been found in patients who do not have the corresponding point mutation, and the exact causes and solutions for the resistance are yet to be solved.

Following imatinib (*i.e.*, a targeting agent for primary CML), secondary targeting agents such as nilotinib, dasatinib, *etc.* have been developed and applied to those patients who have resistance due to the presence of a point mutation. However, there is a need for the development of other strategies to overcome drug resistance.

In recent years, it has been known that the risk for the same cancer-causing factors varies depending on the genetic susceptibility of individuals, and thus, single nucleotide polymorphism (hereinafter, SNP) has been proposed as the most important marker that can distinguish these susceptibilities between individuals and races.

SNP refers to a single base-pair variation in DNA between individuals and is the most common form among the DNA nucleotide sequence polymorphisms (approximately 1 per 1 kb). SNPs can be used as genetic markers on the genome because they are not only a form of a variant occurring most frequently, but also exhibit a fairly stable and low variation rate. This suggests that SNPs provide information on different kinds of diversity that occur in genes. For example, if the SNPs selected through linkage disequilibrium analysis have linkage disequilibrium, the SNPs may serve as an important clue to confirm what diseases might have been caused by changes such as mutations in genes, because the genetic changes that occur within the SNPs also establish the same linkage disequilibrium.

Since this linkage disequilibrium can predict important parts of the recombination of genomic DNA, it can also provide predictable clues to genetic inheritance and genetic diseases in the next generation.

The association between certain types of hematologic malignancies (*e.g*., chronic lymphocytic leukemia, acute lymphoblastic leukemia, myeloid tumors, *etc.)* and genetic mutations has been identified by genome-wide association analysis. Previous studies have shown that the BCL2 gene polymorphism is associated with CML susceptibility for patients with CML.

### [Disclosure]

### [Technical Problem]

The present inventors have studied SNPs which show statistical correlation between a patient group having resistance to the CML targeting agents and a patient group having no resistance to the CML targeting agents, through which drug tolerance of CML patients can be predicted or determined. As a result, the present inventors have found that there is a correlation between the expression levels of particular SNPs within the HMGCLL1 gene and isoform 3 of the HMGCLL1 of CML patients and drug tolerance of the CML patients. The present inventors further have found that the growth of CML cells and malignant tumor cells can be effectively inhibited by inhibiting the expression of the isoform 3 HMGCLL1 (HMGCLL1 IS3).

### [Technical Solution]

An object of the present invention is to provide a pharmaceutical composition for the prevention or treatment of malignant tumors which contains an agent capable of inhibiting the expression of HMGCLL1 IS3.

Another object of the present invention is to provide a pharmaceutical composition for the prevention or treatment of malignant tumors showing resistance to tyrosine kinase inhibitors, which contains an agent inhibiting the expression of HMGCLL1 IS3.

Still another object of the present invention is to provide a method for determining drug resistance in a subject having a malignant tumor using the expression level of HMGCLL1 IS3.

Still another object of the present invention is to provide a composition for diagnosing drug resistance used for the above method.

Still another object of the present invention is to provide a kit for diagnosing drug resistance, which includes the above composition.

Still another object of the present invention is to provide a method for determining the probability of developing a malignant tumor using the expression level of HMGCLL1 IS3

Still another object of the present invention is to provide a method for screening malignant tumor therapeutic agents using the expression level of HMGCLL1 IS3.

Still another object of the present invention is to provide a method for predicting prognosis of malignant tumors using the expression level of HMGCLL1 IS3.

Still another object of the present invention is to provide a composition for predicting the prognosis of malignant tumors, which contains an agent capable of measuring the expression level of HMGCLL1 IS3.

Still another object of the present invention is to provide a kit for predicting the prognosis of malignant tumors including the composition for predicting the prognosis of malignant tumors.

Still another object of the present invention is to provide a method for determining drug resistance in a subject having a malignant tumor, which includes determining the presence of at least one SNP among SEQ ID NOS: 9 to 16 and 33 to 173.

Still another object of the present invention is to provide a composition for diagnosing drug resistance used for the above method.

Still another object of the present invention is to provide a kit for diagnosing drug resistance including the composition for diagnosing drug resistance.

Still another object of the present invention is to provide a method for determining the probability of developing a malignant tumor, which includes determining the presence of at least one SNP among SEQ ID NOS: 9 to 16 and 33 to 173.

Still another object of the present invention is to provide a method for predicting the prognosis of a malignant tumor, which includes determining the presence of at least one SNP among SEQ ID NOS: 9 to 16 and 33 to 173.

Still another object of the present invention is to provide a composition for predicting the prognosis of a malignant tumor, which is used for the above method.

Still another object of the present invention is to provide a kit for diagnosing drug resistance which includes the composition for predicting the prognosis.

### [Advantageous Effects of the Invention]

The features and advantages of the present invention may be summarized as follows.
(a) The present invention provides genes and SNP markers that are associated with drug resistance in patients with CML and malignant tumors.
(b) The present invention provides a kit for diagnosing drug resistance in patients with CML and malignant tumors using the above markers, a method for screening therapeutic agents for treating CML and malignant tumors, and a pharmaceutical composition for the prevention or treatment of CML and malignant tumors.
(c) By using the markers of the present invention, drug tolerance of a patient with chronic myeloid leukemia can be effectively predicted or diagnosed, and furthermore, these markers can be utilized in studies for efficient drug treatment and drug development.

### [Brief Description of Drawings]

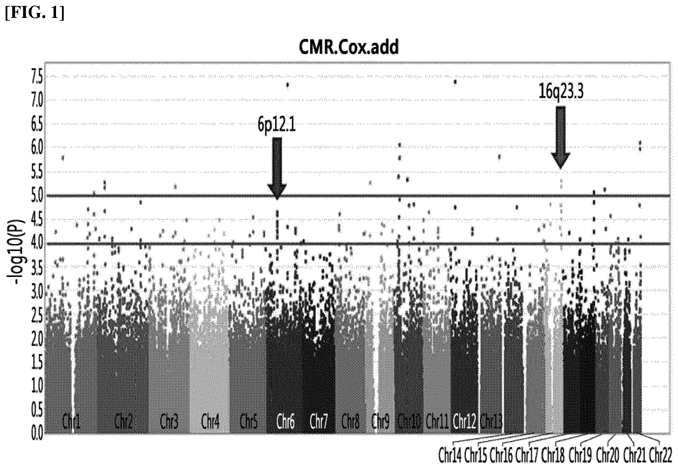

FIG. 1 shows the results of the genome-wide association analysis for CML performed in the Discovery set of the present invention illustrated by the Manhattan plot.

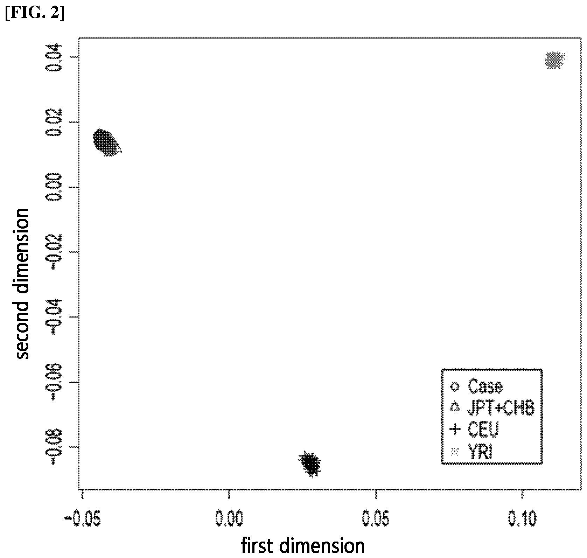

FIG. 2 shows the analysis results of the population structure of samples using the multidimensional scaling (MDS) for the evaluation of stratification after confirming genetic homogeneity.

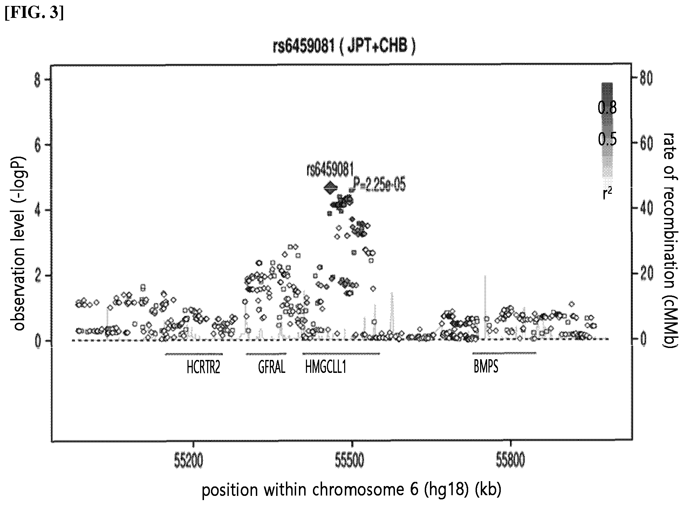

FIG. 3 shows the analysis results of the validation regional association plot in a validation group which belongs to a candidate genetic locus (6p12.1) in the Discovery set.

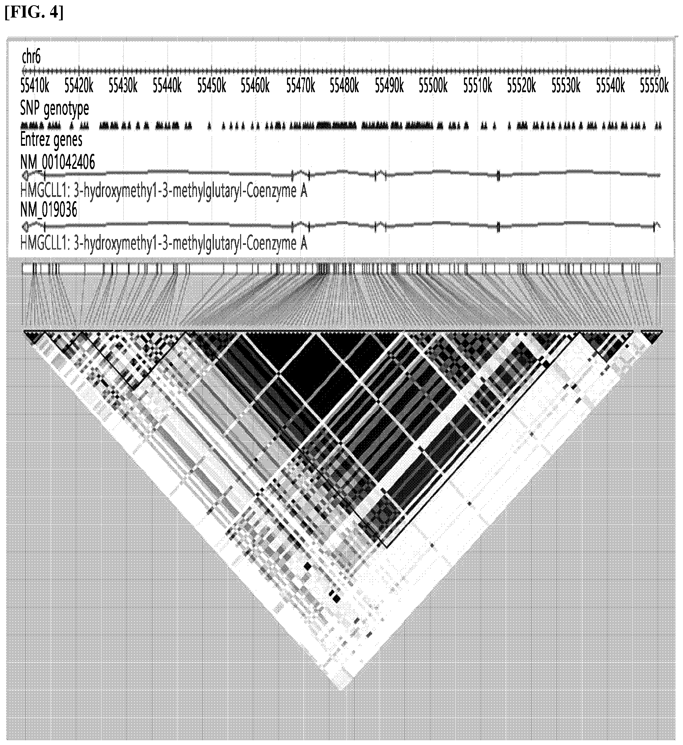

FIG. 4 shows the results corresponding to the linkage disequilibrium (LD) plot with respect to the HMGCLL1 located at the genetic locus 6p12.1. The results are illustrated based on the Hapmap2 release 22 CHB+JPT reference data, and the LD colors were indicated based on r-square.

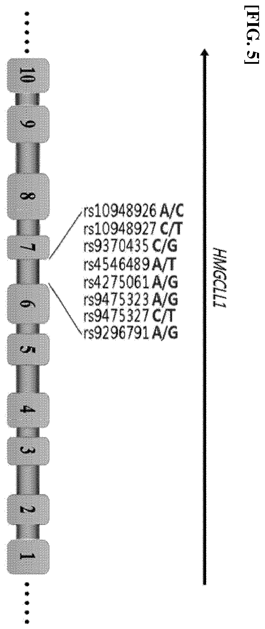

FIG. 5 shows a schematic diagram illustrating each exon of the HMGCLL1 gene and the positions of eight SNPs whose association therewith was validated.
FIGS. 6A and 6B show the results of the plots with respect to the cumulative incidence in Koreans and Westerners, in which FIG. 6A represents the plot with respect to the cumulative incidence in Koreans and FIG. 6B represents the plot with respect to the cumulative incidence in Westerners.
FIG. 7A shows a schematic diagram illustrating the structures of isoforms that can appear in HMGCLL1.
FIG. 7B shows the positions for binding to primers for the quantification of corresponding isoforms and the results of the quantified values.
FIGS. 8A and 8B show the graphs illustrating the comparison results with respect to the effects of HMGCLL1 IS3 siRNA and anticancer targeting agents on the mutated cell line (the K562 cell line which induced the T315I mutation) which show resistance to anticancer targeting agents.
FIGS. 9A to 9C show the graphs illustrating the comparison results with respect to the effects of HMGCLL1 IS3 siRNA and an anticancer targeting agent imatinib on the CML cell line and normal lymphocytes, in which FIG. 9A represents the results of treatment performed in K526 cells (*i.e.,* a CML cell line), FIG. 9B represents the results of treatment performed in CML T1 cells (*i.e.,* a CML cell line), and FIG. 9C represents the results of treatment performed in normal lymphocytes.
FIG. 10 shows the graphs illustrating the comparison results with respect to the changes in the pCrkL/CrkL ratio in K526 cells (*i.e.,* a CML cell line) according to the treatment with HMGCLL1 IS3 siRNA and/or an anticancer targeting agent imatinib.
FIGS. 11A and 11B show the graphs illustrating the comparison results with respect to the changes in the cell cycle in a CML cell line according to the treatment with HMGCLL1 IS3 siRNA, in which FIG. 11A represents the results of treatment performed in K526 cells (*i.e.,* a CML cell line) and FIG. 11B represents the results of treatment performed in LAMA-84 cells (*i.e.,* a CML cell line).
FIGS. 12A to 12C show the graphs illustrating the comparison results with respect to the anticancer effects of HMGCLL1 IS3 siRNA on various cancer cells, in which FIG. 12A represents the results of treatment performed in cancer cell lines of hematopoietic and lymphatic tissue and FIGS. 12B and 12C represent the results of treatment performed in solid cancer cell lines.

### [Best Mode]

The present inventors were able to identify the genetic factors involved in inducing tolerance to anticancer targeting agents with respect to CML. That is, they have confirmed that eight SNPs (*i.e.*, rs10948926 (SEQ ID NO: 9), rs10948927 (SEQ ID NO: 10), rs9370435 (SEQ ID NO: 11), rs4546489 (SEQ ID NO: 12), rs4275061 (SEQ ID NO: 13), rs9475323 (SEQ ID NO: 14), rs9475327 (SEQ ID NO: 15), and rs9296791 (SEQ ID NO: 16)) are involved in the induction of resistance described above. Each of the eight SNPs is located in intron 5 (between exon 6 and exon 7) of the HMGCLL1 gene located on the chromosome 6p12.1.

According to the studies of the present inventors, it was discovered that each of the eight SNPs is related to each other in a mutually dependent manner and that SNPs having the same pattern of homozygous haplotypes exist. According to the studies of the present inventors, the above-mentioned eight SNPs and a total of 141 SNPs of the nucleotide sequences of SEQ ID NOS: 33 to 173 are related to one another in a mutually dependent manner.

Accordingly, in an embodiment of the present invention, there is provided a method for determining the drug resistance of a cancer by determining the presence of SNPs having the sequences represented by SEQ ID NOS: 9 to 16 and 33 to 173. Additionally, in an embodiment of the present invention, there are provided a composition and a kit for detecting the SNPs. The composition and the kit include primer sequences which include nucleotide sequences that can complementarily bind to target sequences in which the SNPs are set as target sequences, probe sequences, and polymerase. In another embodiment of the present invention, there is provided a method for determining an appropriate method for treatment using the information with respect to drug resistance, which has been determined using the above method, composition, and kit.

Additionally, the present inventors have confirmed that the distribution of the haplotypes consisting of the SNPs is significantly associated with the expression of particular isoforms of the HMGCLL1 gene. In particular, they have confirmed that the SNPs are directly associated with the expression of the isoform 3 (IS3, SEQ ID NOS: 2 and 3) of the HMGCLL1.

Accordingly, in an embodiment of the present invention, there is provided a method for determining the presence of resistance to anticancer drugs by measuring the expression level of HMGCLL1 IS3.

According to another embodiment of the present invention, there is provided a method for predicting the prognosis of a cancer by measuring the expression level of HMGCLL1 IS3.

Therefore, appropriate treatment can be proposed to patients according to the results, in which the resistance has been determined using at least one of the above methods. For example, in a case where the sample of a patient diagnosed as having a cancer was subjected to the above methods and determined to have the above SNPs, appropriate treatment for the effective cancer treatment of the patient can be prescribed. For example, the timing of treatment suitable for the use of the primary and secondary anticancer agents can be reasonably determined.

Additionally, surprisingly, the present inventors have confirmed that HMGCLL1 IS3 inhibitors not only exhibit a therapeutic effect on CML in which resistance to anticancer agents was induced, but also on CML in which resistance to anticancer agents was not induced. They have also confirmed, in an experiment using the siRNA of HMGCLL1 IS3 as an inhibitor, that the siRNA of HMGCLL1 IS3 can inhibit the signaling by BCR-ABL1, which induces the development of CML, arresting cell cycles and thereby exhibiting anticancer activity.

Additionally, more surprisingly, the present inventors have confirmed that HMGCLL1 IS3 siRNA exhibits anticancer activity against malignant tumors other than CML. For example, in an experiment using the siRNA of HMGCLL1 IS3 as an inhibitor, HMGCLL1 IS3 siRNA exhibits significant antitumor activity against cancer cells derived from various tissues, such as hematopoietic and lymphoid tissues, autonomic ganglia, biliary tract, breast, central nervous system, liver, pancreas, skin, stomach, urinary system, *etc.*

As such, the effect of exhibiting anticancer activity against various cancer cells by inhibiting the expression of HMGCLL1 IS3 has never been reported until now, and was first found by the present inventors.

According to an embodiment of the present invention, there is provided a pharmaceutical composition for the prevention or treatment of malignant tumors, which contains an agent capable of inhibiting the expression or activity of HMGCLL1 IS3.

### Definition of Terms

As used herein, the term "3-hydroxymethyl-3-methylglutaryl-CoA lyase like 1 (HMGCLL1)" refers to a protein involved in the peroxisome-associated pathway and the butanoate metabolism pathway, or a gene encoding the protein. The HMGCLL1 gene includes ten exon regions and may be expressed in various kinds of isoforms according to the deletion of the exon regions.

The HMGCLL1 is known to have the function of decomposing (S)-3-hydroxy-3-methylglutaryl-CoA into acetyl-CoA and acetoacetate by 3-hydroxymethyl-3-methylglutaryl-CoA lyase outside the mitochondria. Notably, the HMGCLL1 is similar in sequence to HMG-CoA lyase (HMGCL), and it is influential within the mutually complementary pathways with HMG-CoA reductase (HMGCR).

The disease known to be associated with the HMGCL is 3-hydroxy-3-methylglutaryl-CoA lyase (hereinafter, HMGCLL1) deficiency, which is inherited as an autosomal recessive condition. The deficiency of HMG-CoA lyase results in the impairment of ketone formation and causes no or less increase in ketones while inducing hypoglycemia.

The information on the specific nucleotide sequence of the HMGCLL1 gene or the amino acid sequence of the protein is reported in databases such as NCBI (*e.g.,* reported as GenBank Accession Nos. NC_000006.12, NC_000075.6, NC_005107.4, *etc.*)*.* For example, the present invention provides a cDNA of the HMGCLL1 consisting of the nucleotide sequence of SEQ ID NO: 1. In the HMGCLL1 cDNA of SEQ ID NO: 1, the first exon region is a region consisting of the 1^{st} to the 108^{th} nucleotides; the second exon region is a region consisting of the 109^{th} to the 198^{th} nucleotides; the third exon region is a region consisting of the 199^{th} to the 279^{th} nucleotides; the fourth exon region is a region consisting of the 280^{th} to the 387^{th} nucleotides; the fifth exon region is a region consisting of the 388^{th} to the 483^{rd} nucleotides; the sixth exon region is a region consisting of the 484^{th} to the 632^{nd} nucleotides; the seventh exon region is a region consisting of the 633^{rd} to the 696^{th} nucleotides; the eighth exon region is a region consisting of the 697^{th} to the 885^{th} nucleotides; the ninth exon region is a region consisting of the 886^{th} to the 1,011^{th} nucleotides; and the tenth exon region is a region consisting of the 1,012^{th} to the 1,113^{th} nucleotides.

As used herein, the isoform 3 (IS3) of HMGCLL1 is a protein which is expressed in a form where the second and fifth exons in the HMGCLL1 cDNA are deleted. The present inventors first found that HMGCLL1 IS3 performs an important role with respect to the resistance of CML to anticancer agents, and it is possible to treat various kinds of cancers by inhibiting the expression of HMGCLL1 IS3. The information on specific nucleotide sequences or amino acid sequences of the protein for each isoform of the HMGCLL1 gene is reported in databases such as NCBI (*e.g*., GenBank Accession Nos. NP_061909, NP_001035865, NP_001274675, NP_001274670, NP_001274682, NP_001274670, or ENSP00000309737 [ENSEMBL], Q8TB92-5 [UNIPROT], *etc*.).

According to an embodiment of the present invention, the cDNA of HMGCLL1 IS3 has the nucleotide sequence of SEQ ID NO: 2. In the nucleotide sequence of SEQ ID NO: 2, the first exon region is a region consisting of the 1^{st} to the 108^{th} nucleotides; the second exon region (the third exon region of HMGCLL1 cDNA) is a region consisting of the 109^{th} to the 189^{th} nucleotides; the third exon region (the fourth exon region of HMGCLL1 cDNA) is a region consisting of the 190^{th} to the 297^{th} nucleotides; the fourth exon region (the sixth exon region of HMGCLL1 cDNA) is a region consisting of the 298^{th} to the 446^{th} nucleotides; the fifth exon region (the seventh exon region of HMGCLL1 cDNA) is a region consisting of the 447^{th} to the 510^{th} nucleotides; the sixth exon region (the eighth exon region of HMGCLL1 cDNA) is a region consisting of the 511^{th} to the 699^{th} nucleotides; the seventh exon region is a region consisting of the 700^{th} to the 825^{th} nucleotides; and the eighth exon region is a region consisting of the 826^{th} to the 927^{th} nucleotides.

According to an embodiment of the present invention, HMGCLL1 IS3 as a target for the purpose of determining drug resistance, predicting cancer prognosis, and screening anticancer agents, may be one which has a sequence homology to the amino acid sequence of SEQ ID NO: 3, which is expressed from the nucleotide sequence SEQ ID NO: 2, of at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%.

As used herein, the term "agent inhibiting the expression or activity of HMGCLL1 IS3" refers to an agent that reduces the expression or activity of HMGCLL1 IS3 in cancer cells, and the agent may be used for the purpose of preventing or treating cancer development by reducing the expression or activity of HMGCLL1 IS3 in cancer cells. For example, the agent may include compounds, nucleic acids, aptamers, peptides, viruses, vectors which include the nucleic acid, *etc.* that can inhibit the expression or activity of HMGCLL1 IS3. Specifically, the agent may include a polynucleotide capable of suppressing an expression of HMGCLL1 IS3 mRNA, which may include an antisense oligonucleotide, siRNA, RNA aptamer, or the like, or a substance capable of specifically binding the HMGCLL1 IS3 thereby inhibiting an activity of HMGCLL1 IS3, which may include an aptamer, an antibody, a peptide, a fragment of the antibody, a small molecule compound.

As used herein, the term "antisense oligonucleotide or an antisense nucleic acid" refers to DNA or RNA which includes a complementary sequence to a particular mRNA sequence, or a derivative thereof. The antisense oligonucleotide binds to a complementary sequence in the mRNA and acts to inhibit the translation of mRNA into a protein. In embodiments of the present invention, the antisense oligonucleotide may be DNA or RNA which is complementary to the HMGCLL1 mRNA and is able to bind to the HMGCLL1 mRNA. The antisense oligonucleotide may inhibit the translation of the HMGCLL1 mRNA, translocation into the cytoplasm, maturation, or other essential activities with respect to general biological functions. In an embodiment, the length of the antisense oligonucleotide may be 6 to 100 nucleotides, in another embodiment, may be 8 to 60 nucleotides, and in still another embodiment, may be 10 to 40 nucleotides, but the length of the antisense oligonucleotide is not particularly limited thereto.

The antisense nucleic acid may be modified in at least one position of a base, sugar, or backbone for improving the effect thereof (De Mesmaeker et al., Curr Opin Struct Biol., 5(3): 343 to 355 (1995)). The nucleic acid backbone may be modified by phosphorothioate, phosphotriester, methylphosphonate, short-chain alkyl, cycloalkyl, short-chain heteroatomic, heterocyclic bonds, *etc.* Additionally, the antisense nucleic acid may include at least one substituted sugar moiety. The antisense nucleic acid may include a modified nucleotide. Examples of modified bases may include hypoxanthine, 5-methyladenine, 5-methylpyrimidine (in particular 5-methylcytosine), 5-hydroxymethylcytosine (HMC), glycosyl HMC, gentobiosyl HMC, 2-aminoadenine, 2-thiouracil, 2-thiothymine, 5-bromouracil, 5-hydroxymethyluracil, 8-azaguanine, 7-deazaguanine, N6 (6-aminohexyl) adenine, 2,6-diaminopurine, *etc.* Additionally, the antisense nucleic acid of the present invention may chemically bind to at least one moiety or conjugate which can improve the activity and cell adsorption of the antisense nucleic acid. Examples of a fat-soluble moiety may include cholesterol moiety, cholesteryl moiety, cholic acid, thioether, thiocholesterol, fatty chain, phospholipid, polyamine, polyethylene glycol chain, adamantane acetic acid, palmityl moiety, octadecylamine, hexylamino-carbonyl-oxycholesterol moieties, *etc.,* but the fat-soluble moiety is not limited thereto. The method for manufacturing oligonucleotides including the fat-soluble moiety is already well known in the art to which the present invention belongs (U.S. Pat. Nos. 5,138,045, 5,218,105, and 5,459,255). The modified nucleic acid can increase stability to nucleases and increase the binding affinity between an antisense nucleic acid and a target mRNA.

An antisense oligonucleotide may be synthesized *in vitro* by a conventional method and administered to the living body or it may synthesized *in vivo.* In an embodiment, an antisense oligonucleotide may be synthesized *in vitro* using RNA polymerase I. In an embodiment, an antisense oligonucleotide may be synthesized *in vivo* by transcribing an antisense RNA using a vector which has the origin of the recognition site (MCS) on the opposite direction. It is preferred that the antisense RNA be allowed to have a translation stop codon within the sequence so that the antisense RNA cannot be translated into a peptide sequence.

As used herein, the term "siRNA" refers to a nucleic acid molecule that can cause RNA interference or mediate gene silencing (see WO 00/44895, WO 01/36646, WO 99/32619, WO 01/29058, WO 99/07409, and WO 00/44914). Since siRNA can inhibit the expression of a target gene, it is provided in a method for efficient gene knockdown or gene therapy. The siRNA was first discovered in plants, worms, fruitflies, and parasites, and it is recently used for the study of mammalian cells. Usually, when molecules are used, the siRNA may have a double-stranded structure, in which a sense strand (a sequence corresponding to the HMGCLL1 mRNA sequence) and an anti-sense strand (a sequence complementary to the HMGCLL1 mRNA sequence) are positioned at sides opposite to each other; or may have a single-stranded structure in which a self-complementary sense strand or anti-sense strand is present. The siRNA is not limited to those in which double-stranded RNA parts form a perfect pair between RNAs, but it may include RNA parts which do not form a pair by a mismatch (the corresponding nucleotides are not complementary), a bulge (absence of nucleotides corresponding to the chain of one party), *etc.* Additionally, siRNAs with a structure of a blunt end and a sticky end are possible as long as they can inhibit the expression of HMGCLL1 gene by the RNAi effect. As the sticky end structure, both the 3'-end protruding structure and the 5'-end protruding structure are possible. The siRNA molecule may have a shape in which a short nucleotide sequence (e.g., about 5 nt to 15 nt) between a self-complementary sense and anti-sense strands. In this case, the siRNA molecule formed by the expression of a nucleotide sequence forms a hairpin structure by intramolecular hybridization and overall forms a stem-and-loop structure. The stem-and-loop structure is processed *in vivo* or *in vitro* and thereby forms an active siRNA molecule that can mediate RNAi.

In embodiments of the present invention, the siRNA may be those which can bind to the mRNA of HMGCLL1 IS3. In an embodiment, the siRNA may be one which comprises a nucleotide sequence that can specifically bind to a part or the entirety of a region (a region of the 190^{th} to the 446^{th} nucleotides of SEQ ID NO: 2) derived from the third exon (the fourth exon of HMGCLL1 cDNA) and the fourth exon (the sixth exon of HMGCLL1 cDNA) of the cDNA of HMGCLL1 IS3 of SEQ ID NO: 2; and in another embodiment, the siRNA may be one which comprises a nucleotide sequence that can specifically bind to a part or the entirety of a region derived from the nucleotide sequence of SEQ ID NO: 4, which is included in the linking region between the third exon and the fourth exon of HMGCLL1 IS3 cDNA of SEQ ID NO: 2; and in still another embodiment, the siRNA may be one which comprises nucleotide sequences of SEQ ID NOS: 5 to 8, but the sequences of siRNA are not particularly limited thereto.
SEQ ID NO: 4: 
5′-AGAAGTGACTAGCTTTGTGTCTTCCAGATGGGTACCACAGGTTGCTGCTGGAGCT
ACTGAGATATCAGTTTTTGGAGCTG-3′
SEQ ID NO: 5: 5'-AGCAGCAACCUGUGGUACC-3'
SEQ ID NO: 6: 5'-AACCUGUGGUACCCA-3'
SEQ ID NO: 7: 5'-GCAGCAACCUGUGGU-3'
SEQ ID NO: 8: 5'-CUCCAGCAGCAACCU-3'

In still another embodiment, the siRNA may be one which includes any one nucleotide sequence selected from the group consisting of SEQ ID NOS: 5 to 8, and includes 19 to 40 continuous nucleotides that can specifically bind to a part or the entirety of the nucleotide sequence of SEQ ID NO: 4.

As used herein, the term "RNA aptamer" refers to a nucleic acid ligand molecule which binds to a target mRNA by the ability of adopting a particular 3-dimensional stereoscopic shape, thereby having an antagonistic effect on the same. Typically, aptamers may be small nucleic acids with a length of 15 to 50 nucleotides that fold into defined secondary and tertiary structures, such as a stem-loop structure. It is preferred that aptamers bind to a target molecule having a K_{d} less than 10⁻⁶, 10⁻⁸, 10⁻¹⁰, or 10⁻¹². Aptamers can bind to the target molecule with a significantly high degree of specificity. Additionally, aptamers have been isolated that have greater than a 10,000-fold difference in binding affinities between the target molecule and another molecule that differ at only a single position on the molecule. Aptamers may be comprised of multiple ribonucleotide units, deoxyribonucleotide units, or a mixture of both types of nucleotide residues. Aptamers may further include one or more modified bases, sugars, or phosphate backbone units. Aptamers are an oligonucleotide material (*e.g*., RNA) or a peptide material and are described in detail in the literature (Bock LC et al., Nature 355 (6360): 5646 (1992); Hoppe-Seyler F, Butz K "Peptide aptamers: powerful new tools for molecular medicine". J Mol Med. 78(8): 42630 (2000); Cohen BA, Colas P, Brent R. "An artificial cell-cycle inhibitor isolated from a combinatorial library". Proc Natl Acad Sci U.S.A.. 95(24): 142727 (1998)).

As used herein, the term "antibody" refers to a material which responds to the invasion of an antigen (*i.e.,* a foreign material) while circulating in the blood or lymph in the immune system of a living body. Antibodies are globulin proteins formed in the lymphatic tissue and also called immunoglobulin. Antibodies, which are proteins produced by B cells and secreted into the body fluids, specifically bind to antigens. One molecule of antibody has two heavy chains and two light chains, and each heavy chain and light chain has a variable region at the N-terminal end. Each variable region consists of three complementarity-determining regions (CDRs) and four framework regions (FRs), in which the CDRs determine the antigen-binding specificities of antigens and are present in relatively short peptide sequences that are maintained by the FRs in the variable region.

In embodiments of the present invention, the antibodies may be polyclonal or monoclonal antibodies that specifically bind to HMGCLL1 IS3, thereby inhibiting the activity thereof. The antibodies to the HMGCLL1 IS3 may be prepared using the methods commonly used in the art (*e.g.,* a fusion method (U.S. Pat. No. 4,816,56) or a phage antibody library method (Clackson et al., Nature, 352: 624 to 628 (1991) and Marks et al., J. Mol. Biol., 222: 58, 1 to 597 (1991)). General processes with respect to antibody preparation are described in detail in the literature (Harlow, E. and Lane, D., Using Antibodies: A Laboratory Manual, Cold Spring Harbor Press, New York, 1999; Zola, H., Monoclonal Antibodies: A Manual of Techniques, CRC Press, Inc., Boca Raton, Florida, 1984; and Coligan, CURRENT PROTOCOLS IN IMMUNOLOGY, Wiley/Greene, NY, 1991), and these are incorporated herein by reference. For example, the preparation of hybridoma cells producing monoclonal antibodies can be achieved through a fusion between an immortalized cell line and antibody-producing lymphocytes, and the technique necessary for this process is well known to one of ordinary skill in the art and can easily be performed. The polyclonal antibodies can be obtained by a method which includes administration of an HMGCLL1 IS3 antigen to an appropriate animal, collection of the antiserum from the animal, and isolation of the antibodies from the antiserum using a known affinity technology.

The peptides which can specifically bind to HMGCLL1 IS3 and inhibit the activity of HMGCLL1 IS3 can be obtained by a method known in the art, *e.g.,* a phage display method (Smith GP, "Filamentous fusion phage: novel expression vectors that display cloned antigens on the virion surface". Science 228 (4705): 1315 to 1317 (1985); Smith GP, Petrenko VA, "Phage display". Chem. Rev. 97(2): 391 to 410 (1997)).

As used herein, the term "malignant tumor", which is a disease associated with the regulation of cell death, refers to a disease caused by excessive cell proliferation when the balance in normal cell death is broken. These abnormal hyperproliferative cells sometimes invade surrounding tissues and organs to form a mass or destroy or alter the normal structures of the body, which is called a malignant tumor or cancer. Generally, a tumor refers to a mass that is abnormally grown by autonomous overgrowth of the body tissue, and may be divided into benign tumors and malignant tumors. Malignant tumors have a much faster growth rate than benign tumors and cause metastasis as they invade the surrounding tissues, thus threatening life.

In embodiments of the present invention, the malignant tumors may not be particularly limited as long as the malignant tumors can be treated by inhibiting the expression or activity of HMGCLL1 IS3. In an embodiment, the malignant tumors may be those derived from hematopoietic tissue or lymphatic tissue, solid cancer, *etc.* In another embodiment, the malignant tumors may be those derived from various tissues such as hematopoietic tissue or lymphatic tissue, autonomic ganglia, biliary tract, breasts, central nervous system, livers, pancreas, skin, stomach, urinary system, *etc.*

In particular, the malignant tumors according to the present invention may be those which exhibit resistance to tyrosine kinase inhibitors, and in addition, those which are mutated to exhibit resistance to anticancer agents during the course of treatment with various anticancer agents.

As used herein, the term "prevention" refers to all kinds of actions associated with the inhibition or delay of the development of malignant tumors by administering the pharmaceutical composition for the prevention or treatment of malignant tumors according to the present invention.

As used herein, the term "treatment" refers to all kinds of actions associated with the improvement of malignant tumors such as delaying or arresting the growth of malignant tumors or advantageous changes such as converting cells which are progressing toward a malignant tumor into normal cells by administering the pharmaceutical composition for the prevention or treatment of malignant tumors according to the present invention.

The pharmaceutical composition may further contain an appropriate carrier, excipient, or diluent which is conventionally used in the preparation of pharmaceutical compositions, and the carrier may be a non-natural carrier. Examples of the carrier, excipient, or diluent may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.*

Meanwhile, the pharmaceutical composition of the present invention may be formulated in the form of powders, granules, tablets, capsules, suspensions, emulsions, syrups, aerosols, *etc.* for oral administration; agents for external use; suppositories; and sterile injection solutions according to each conventional method. The formulations may be prepared using a commonly used diluent or excipient such as a filler, extender, binder, humectant, disintegrant, surfactant, *etc.* Solid formulations for oral administration may include tablets, pills, powders, granules, capsules, *etc.,* and these solid formulations may be prepared by adding at least one excipient (*e.g.,* starch, calcium carbonate, sucrose or lactose, gelatin, *etc*.). Additionally, lubricants such as magnesium stearate, talc, *etc.* may be used in addition to the simple excipient. Liquid formulations for oral administration may include suspensions, liquid medicine for internal use, emulsions, syrups, *etc.,* and various excipients such as humectants, sweeteners, fragrances, preservatives, *etc.* may be used in addition to the simple diluents such as water and liquid paraffin. Formulations for parenteral administration may include sterile aqueous solutions, non-aqueous solvents, suspensions, emulsions, lyophilized formulations, suppositories, *etc.* Examples of the non-aqueous solvents and suspensions may include vegetable oils such as propylene glycol, polyethylene glycol, and olive oil; an injectable ester such as ethyl oleate, *etc.* Examples of the bases for suppositories may include Witepsol, macrogol, Tween 61, cacao butter, laurinum, glycerogelatin, *etc.*

The pharmaceutical composition may be prepared in any formulation selected from the group consisting of tablets, pills, powders, granules, capsules, suspensions, liquid medicine for internal use, emulsions, syrups, sterile injection solutions, non-aqueous solvents, lyophilized formulations, and suppositories.

HMGCLL1 IS3 inhibitor may be contained in the pharmaceutical composition of the present invention in an amount of 0.0001 wt% to 50 wt% relative to the total weight of the final composition, and preferably 0.001 wt% to 10 wt%, but the amount of HMGCLL1 IS3 inhibitor is not particularly limited thereto.

The pharmaceutical composition of the present invention may be administered in a pharmaceutically effective amount. As used herein, the term "pharmaceutically effective amount" refers to an amount sufficient for the treatment of diseases at a reasonable benefit/risk ratio applicable to medical treatment, and the level of the effective dose may be determined based on the factors including the kind of a subject, severity of illness, age, sex, drug activity, drug sensitivity, duration of administration, administration route and dissolution rate, duration of treatment, factors including drugs to be used simultaneously in combination, and other factors well known in the medical field. The pharmaceutical composition of the present invention may be administered as an individual therapeutic agent, in combination with other therapeutic agents, or sequentially or simultaneously with conventional therapeutic agents, and may be administered once or multiple times. It is important to administer the pharmaceutical composition in an amount to obtain the maximum effect with a minimum amount without adverse effects considering all of the factors described above.

The dose of the composition of the present invention can be determined by one of ordinary skill in the art in consideration of the purpose of use, addicted level of a disease, age, weight, sex, and medical history of a patient, kinds of materials used as active ingredients, *etc.* For example, the pharmaceutical composition of the present invention may be administered in an amount of about 0.1 ng/kg to about 100 mg/kg (body weight), and preferably about 1 ng/kg to about 10 mg/kg. The pharmaceutical composition of the present invention may be administered once daily or in a few divided doses per day, but the frequency of administration is not particularly limited thereto.

In another aspect, the present invention provides a method for preventing or treating a malignant tumor, which includes administering the pharmaceutical composition to a subject excluding humans having developed or having the potential of developing a malignant tumor.

As used herein, the term "subject" includes animals such as horses, sheep, pigs, goats, camels, antelopes, dogs, *etc.,* or humans in which a malignant tumor has developed or there is a high potential of developing a malignant tumor and in which the symptoms thereof can be improved by the administration of the composition according to the present invention. Malignant tumors can be effectively prevented and treated by administering the composition according to the present invention to a subject. The above method for treatment may be a method for treating animals excluding humans, but the subject to be treated is not limited thereto. That is, considering that the symptoms of malignant tumors in humans can be improved by the administration of the composition for treatment according to the present invention, the pharmaceutical composition of the present invention can certainly be used for the treatment of humans.

As used herein, the term "administration" refers to the introduction of the pharmaceutical composition of the present invention to a subject by any appropriate method. The pharmaceutical composition of the present invention may be administered via various oral and parenteral routes as long as the pharmaceutical composition can arrive at a target tissue.

The pharmaceutical composition may be appropriately administered to a subject according to the conventional method, route of administration, and dose used in the art depending on the purpose or necessity. Examples of the administration routes may include oral, parenteral, subcutaneous, intraperitoneal, intrapulmonary, and intranasal administrations, and the parenteral administration may include intramuscular, intravenous, intraarterial, intraperitoneal, and subcutaneous administrations. Additionally, an appropriate dose and the number of administration may be selected according to the methods known in the art, and the dose and the frequency of administration of the pharmaceutical composition of the present invention actually administered may be appropriately determined by various factors such as the type of the symptom to be treated, administration route, sex, health conditions, diet, age and weight of a subject, and severity of the disease.

As used herein, the term "pharmaceutically effective amount" refers to an amount sufficient for the inhibition or alleviation of vascular permeability at a reasonable benefit/risk ratio applicable to medical treatment, and the level of the effective dose may be determined based on the factors including the kind of a subject, severity of illness, age, sex, drug activity, drug sensitivity, duration of administration, administration route and dissolution rate, duration of treatment, factors including drugs to be used simultaneously in combination, and other factors well known in the medical field. The composition of the present invention may be administered as an individual therapeutic agent, in combination with other therapeutic agents, or sequentially or simultaneously with conventional therapeutic agents, and may be administered once or multiple times. It is important to administer the pharmaceutical composition in an amount to obtain the maximum effect with a minimum amount and without adverse effects considering all of the factors described above, and the pharmaceutically effective amount can easily be determined by one of ordinary skill in the art.

In still another aspect, the present invention provides a method for determining drug resistance in a subject having a malignant tumor.

Specifically, the method for determining drug resistance in a subject having a malignant tumor includes (a) measuring the expression level of HMGCLL1 IS3 from a biological sample isolated from a subject in which a malignant tumor has developed; and (b) comparing the measured expression level of HMGCLL1 IS3 with that of a control sample from a healthy subject or from a patient who does not develop resistance.

In the above method, when the expression level of HMGCLL1 IS3 measured in (a) is higher than that of a sample of the control group, the subject may be determined to have drug resistance to the malignant tumor. Drugs may include an anticancer agent, in particular a kinase inhibitor which is or may be used to treat a malignant cancer, in particular cancers involving at least one mutation in oncogenes such as a BCR-ABL mutation. Kinase inhibitors can be a tyrosine kinase inhibitor, which currently is used as a part of standard treatment or under clinical trials and includes, but not is limited to, imatinib, nilotinib, dasatinib, and the like.

According to an embodiment of the present invention, the present inventors have preformed studies on SNPs which show a statistical correlation between a group of patients having drug resistance to a CML targeting agent and a group of patients having no drug resistance to a CML targeting agent, and have attempted to predict or diagnose the drug tolerance of a CML patient based on the results of the study. As a result, the present inventors have identified that there is a correlation between particular SNPs in the HMGCLL1 gene and the expression level of HMGCLL1 IS3, and they have confirmed that the growth of CML cells and malignant tumor cells is effectively inhibited when the expression of the HMGCLL1 IS3 is inhibited.

Additionally, the genome-wide association analysis was performed for a total of 474 subjects (202 Korean patients and 272 Westerner patients) who were clinically diagnosed with CML (see Table 1). Cox's proportional hazard additive model was used as a statistical method. As a response index to drug response, the CML patients were administered with imatinib and then divided into a group with resistance to imatinib and a group without resistance to imatinib depending on whether each patient had reached the complete molecular response. As a result, it was found that the gene which specifically appears in the imatinib-resistance group is the HMGCLL1 gene present on chromosome No. 6, and that this region is a novel gene region which had not been known to be associated with drug resistance. In particular, genotyping was performed for SNPs located in two candidate regions (chromosome Nos. 6 and 16), and as a result, the eight SNPs of rs10948926, rs10948927, rs9370435, rs4546489, rs4275061, rs9475323, rs9475327, and rs929679 present on chromosome No. 6 were finally drawn and these SNPs were confirmed to be associated with drug resistance of CML patients (see Table 3). Furthermore, a total of 141 SNPs of SEQ ID NOS: 33-173, having a homozygous haplotype with the same pattern as that of the 8 SNPs above were further identified, and this result suggests that the 141 SNPs are in high linkage disequilibrium (LD) relationship (r²>0.8) with the 8 SNPs and they are highly correlated with each other. This suggests that the 141 SNPs, which show the same haplotype pattern as that of the 8 SNPs, can also have an effect on the target gene, HMGCLL1.

The genome-wide association analysis of the present invention has not only evaluated drug tolerance in as many CML patients as possible, but has also included the evaluation of Koreans and European-Canadians, thereby confirming the significance present between different races.

It was found that the ACGTAATG haplotype of the SNPs elucidated by the present inventors is significantly correlated with a drug response in the CML patient group and that the distribution of these haplotypes is significantly correlated with the expression of a particular isoform of the corresponding gene. Furthermore, it was confirmed by experiments that the expression level of HMGCLL1 IS3 is correlated with the CML drug resistance.

The drugs used in the present invention for determining drug resistance of CML patients include known kinase inhibitors such as imatinib, nilotinib, and dasatinib, which are therapeutic agents for CML, but the drugs are not limited thereto.

As used herein, the term "drug tolerance" refers to a lower level of reactivity to a particular drug administered in a certain amount to an organism compared to that in a normal group. The term "drug tolerance" is sometimes interchangeably used throughout the specification with the term "drug resistance," which is development of the ability to withstand the previously destructive effect of a drug by microorganisms or tumor cells.

The expression level of HMGCLL1 IS3 in a biological sample can be identified by examining the amount of mRNA or a protein thereof. The process of isolating mRNAs and proteins may be performed using a known process and the amount of mRNAs and proteins may be measured by various methods.

As used herein, the term "biological sample" refers to a body fluid, including urine, saliva, blood, blood plasma, serum, *etc.,* which can be easily collected and used for the detection of the level of mRNA or a protein of HMGCLL1 IS3.

As used herein, the term "measurement of mRNA level" refers to a process for confirming the presence of mRNA of HMGCLL1 IS3 and the expression level thereof in a biological sample for the detection of HMGCLL1 IS3, whereby the amount of mRNA is measured. Examples of the methods for their analysis may include RT-PCR, competitive RT-PCR, real-time RT-PCR, RNase protection assay, northern blotting, DNA chip, *etc.,* but the method is not limited thereto.

Through these detection methods, the mRNA level of the patients in the control group not showing drug tolerance and that of the subject can be compared, and the presence of the subject's tolerance to CML drugs can be diagnosed by determining the presence of a significant increase in the expression level of mRNA in HMGCLL1 IS3.

As used herein, the term "measurement of protein level" refers to a process of confirming the presence of a protein expressed from HMGCLL1 IS3 in a biological sample and the expression level of the protein, and in an embodiment, the expression level of the protein can be confirmed using an antibody that specifically binds to the protein of the gene.

Examples of the methods for the analysis of the protein level using antibodies may include western blotting, enzyme linked immunosorbent assay (ELISA), radioimmunoassay (RIA), radioimmunodiffusion, Ouchterlony immunodiffusion, rocket electrophoresis, histoimmunological staining, immunoprecipitation, complement fixation assay, FACS, and protein chips, but the analysis methods are not limited thereto.

In still another aspect, the present invention provides a composition for diagnosing drug resistance which can be used in the method for determining drug resistance of the subject having a malignant tumor. Specifically, the composition may contain an agent capable of measuring the expression level of HMGCLL1 IS3.

As used herein, the term "agent capable of measuring the expression level of HMGCLL1 IS3" refers to a means which can be used for the amplification or detection of the gene or can specifically bind to the protein to be used for the detection for the purpose of confirming or measuring the expression level of HMGCLL1 IS3 gene or protein thereof, for example, a primer, a probe, an aptamer, an antibody, a peptide, a fragment of the antibody, a low molecular weight compound, *etc.*

As used herein, the term "primer" refers to a nucleic acid sequence having a short free 3' hydroxyl group, which can form a base pair with a complementary template and function as a starting point for the replication of a template strand.

A primer can initiate DNA synthesis in the presence of reagents and four kinds of nucleoside triphosphates for polymerization (*i.e.*, DNA polymerase or reverse transcriptase) in an appropriate buffer at an appropriate temperature. PCR conditions and the length of sense and antisense primers can be controlled based on the disclosure in the art.

In an embodiment of the present invention, the primer may be a primer pair consisting of the nucleotide sequences of SEQ ID NOS: 174 and 175, but the primer sequence is not particularly limited thereto as long as it can amplify HMGCLL1 IS3 gene by PCR.

As used herein, the term "probe" refers to a fragment of a nucleic acid such as RNA or DNA corresponding to a few nucleotides to a few hundred nucleotides. The probe may be prepared in the form of an oligonucleotide probe, single-stranded DNA probe, double-stranded DNA probe, RNA probe, *etc.* and may be labeled for easier detection.

In the present invention, the nucleotide sequence of the probe is not particularly limited as long as it can complementarily bind to a part or the entirety of HMGCLL1 IS3 gene and thereby detect HMGCLL1 IS3 gene, as is the case with the primer described above.

In still another aspect, the present invention provides a kit for diagnosing drug resistance which includes the composition for diagnosing drug resistance.

The kit according to an embodiment of the present invention may be in a form of microarray. When the kit is in a form of microarray, probes are immobilized on the solid-phase surface of the microarray.

The probes used in the kit according to an embodiment of the present invention have sequences complementary to 10 to 100 continuous nucleotide sequences in each gene including the SNPs of the present invention listed above.

The list of nucleotide sequences of the marker according to an embodiment of the present invention to be referred to in the preparation of the probes can be confirmed by GenBank. For example, with respect to the regions of the SNPs, which are the target markers, probes can be designed by referring to the nucleotide sequences described in the GenBank SNP database (*i.e.*, rs10948926 (SEQ ID NO: 9), rs10948927 (SEQ ID NO: 10), rs9370435 (SEQ ID NO: 11), rs4546489 (SEQ ID NO: 12), rs4275061 (SEQ ID NO: 13), rs9475323 (SEQ ID NO: 14), rs9475327 (SEQ ID NO: 15), and rs9296791 (SEQ ID NO: 16)) and the GenBank SNP database sequences described in Table 6 of Example 5.

In the microarray according to an embodiment of the present invention, the probes are used as a hybridizable array element and are immobilized on a substrate. The preferred support is an appropriate rigid or semi-rigid support and includes, for example, membranes, filters, chips, slides, wafers, fibers, magnetic or non-magnetic beads, gels, tubing, plates, polymers, microparticles, and capillaries. The hybridizable array elements are arranged and immobilized on the substrate. Such immobilization is performed by a chemical bonding method or a covalent bonding method such as UV. For example, the hybridizable array elements may be bonded to a glass surface modified so as to include an epoxy compound or aldehyde group, and additionally, may be bonded on a polylysine-coated surface by UV. Additionally, the hybridizable array element may be bonded to a support through a linker (*e.g*., ethylene glycol oligomer and diamine).

Meanwhile, the sample DNA to be applied in the microarray according to an embodiment of the present invention may be labeled and is hybridized with array elements on the microarray. Hybridization can be performed under various conditions. The detection and analysis of hybridization degree can be variously performed depending on the labeling materials.

The labeling of a probe can provide a signal to detect the presence of hybridization, and the labeled probe be linked to an oligonucleotide. Suitable labels may include fluorophores (*e.g*., fluorescein, phycoerythrin, rhodamine, lissamine, and Cy3 and Cy5 (Pharmacia)), chromophores, chemiluminescers, magnetic particles, radioisotopes (P³² and S³⁵), mass labels, electron-dense particles, enzymes (alkaline phosphatase or horseradish peroxidase), co-factors, substrates for enzymes, heavy metals (*e.g.,* gold), and haptens having a specific binding partner (*e.g*., antibodies, streptavidin, biotin, digoxigenin, and chelating groups). Labeling may be performed according to various conventional methods known in the art, such as nick translation, a random priming method (Multiprime DNA labeling systems booklet, "Amersham" (1989)), and a kination method (Maxam & Gilbert, Methods in Enzymology, 65: 499 (1986)). The labels provide signals that can be detected by fluorescence, radioactivity, measurement of color development, weight measurement, x-ray diffraction or absorption, magnetic force, enzymatic activity, mass analysis, binding affinity, high frequency hybridization, or nanocrystals.

The nucleic acid samples for analysis may be prepared using mRNAs obtained from various biosamples. Instead of probes, cDNA may be labeled for hybridization-based analysis.

In a case where probes are used, the probes will be hybridized with cDNA molecules. In the present invention, appropriate hybridization conditions may be determined through a series of processes by an optimization procedure. The procedure is performed in a series of processes by one of ordinary skill in the art for the establishment of protocols for laboratory use. For example, conditions such as temperature, concentration of components, hybridization and washing times, buffer components, and their pH and ionic strength may vary depending on various factors (*e.g.,* length and GC content of probes, target nucleotide sequence, *etc*.). The specific conditions for hybridization can be found in Joseph Sambrook, et al., Molecular Cloning, A Laboratory Manual, Cold Spring Harbor Laboratory Press, Cold Spring Harbor, N.Y. (2001); and M. L. M. Anderson, Nucleic Acid Hybridization, Springer-Verlag New York Inc. N.Y. (1999). For example, the high stringent condition refers to performing a hybridization in 0.5 M NaHPO₄, 7% sodium dodecyl sulfate (SDS), and 1 mM EDTA at 65°C followed by washing in 0.1× standard saline citrate (SSC)/0.1% SDS at 68°C. Alternatively, the highly stringent condition may refer to washing in 6× SSC/0.05% sodium pyrophosphate at 48°C. The less stringent condition refers to, for example, washing in 0.2× SSC/0.1% SDS at 42°C.

After the hybridization reactions, hybridization signals generated through the hybridization reactions are detected. The detection of the hybridization signals may be analyzed by various methods depending on the labels bound to the probes. For example, when probes are labeled with enzymes, the presence of hybridization may be detected by reacting substrates for enzymes with hybridization resultants. Examples of the enzyme-substrate combination to be used in the present invention may include a combination of peroxidase (e.g., horseradish peroxidase) and a substrate of chloronaphtol, aminoethylcarbazol, diaminobenzidine, D-luciferin, lucigenin (bis-*N*-methylacridinium nitrate), resorufin benzyl ether, luminol, Amplex Red reagent (10-acetyl-3,7-dihydroxyphenoxazine), *p*-phenylenediamine-HCl and pyrocatechol (HYR), 3,3',5,5'-tetramethylbenzidine (TMB), 2,2'-azino-di[3-ethylbenzthiazoline sulfonate] (ABTS), o-phenylenediamine (OPD), or naphthol/pyronine; a combination of alkaline phosphatase and a substrate of bromochloroindolylphosphate (BCIP), nitro blue tetrazolium (NBT), or naphthol-AS-Bl-phosphate or ECF; and a combination of glucosidase and a substrate of *t*-nitroblue tetrazolium (NBT) or *m*-phenazine methosulfate (PMS), *etc.* When the probes are labeled with gold particles, the presence of hybridization may be detected by silver staining method using silver nitrate. Accordingly, when the kit of the present invention is used based on the hybridization, specifically, the steps of (i) hybridizing a probe having a nucleotide sequence complementary to the nucleotide sequence of the marker of the present invention; and (ii) detecting the occurrence of hybridization may be included. The drug tolerance of CML patients can be determined by analyzing the intensities of the hybridization signals by the hybridization process. That is, in a case where the hybridization signal with respect to the nucleotide sequence of the target marker of the present invention is shown to be stronger compared to that of the normal sample, it is determined that the patient has resistance to CML drugs.

In still another aspect, the present invention provides a method for determining the probability of developing a malignant tumor.

Specifically, the method for determining the probability of developing a malignant tumor includes (a) measuring the expression level of HMGCLL1 IS3 from a biological sample isolated from a subject who is suspected to have a malignant tumor has developed; and (b) comparing the measured expression level of HMGCLL1 IS3 with that of a control sample which is determined to be free of the malignant tumor.

In the above method, when the expression level of HMGCLL1 IS3 measured in (a) is higher than that of a sample isolated from a control subject, the suspected subject is determined to have a higher probability of developing a malignant tumor than the control subject.

In still another aspect, the present invention provides a method for screening malignant tumor therapeutic agents.

Specifically, the method for screening malignant tumor therapeutic agents includes (a) treating an isolated malignant tumor cell sample which expresses HMGCLL1 IS3, with a candidate material of a malignant tumor therapeutic agent; and (b) comparing the expression level of HMGCLL1 IS3 measured in a sample treated with a candidate material with that of HMGCLL1 IS3 measured in the control sample not treated with the candidate material.

As used herein, the term "candidate material" refers to a material, which is to be tested for an action of reducing the expression of HMGCLL1 IS3 or action of treating a malignant tumor and thereby exhibiting an effect of reducing the expression level of HMGCLL1 IS3. Examples of the candidate material may include a chemical material, peptide, nucleotide, antisense-RNA, and small interference RNA (siRNA), but the candidate material is not limited thereto.

The test material to be evaluated by the screening method of the present invention may be a single compound or a mixture of compounds. The test material may be obtained from a library of synthetic or natural compounds. The methods for obtaining libraries of these compounds are known in the art. Libraries of synthetic compounds are commercially available from Maybridge Chemical Co. (U.K.), Comgenex (U.S.A.), Brandon Associates (U.S.A.), Microsource (U.S.A.), and Sigma-Aldrich (U.S.A.), and libraries of natural compounds are commercially available from Pan Laboratories (U.S.A.) and MycoSearch (U.S.A.).

Test materials may be obtained by various combinatorial library methods known in the art, for example, biological libraries, spatially addressable parallel solid-phase or solution-phase libraries, synthetic library methods requiring deconvolution; the "one-bead one-compound" library method; and synthetic library methods using affinity chromatography selection. Methods for the synthesis of molecular libraries are disclosed in DeWitt et al., Proc. Natl. Acad. Sci. U.S.A. 90, 6909, 1993; Erb et al. Proc. Natl. Acad. Sci. U.S.A. 91, 11422, 1994; Zuckermann et al., J. Med. Chem. 37, 2678, 1994; Cho et al., Science 261, 1303, 1993; Carell et al., Angew. Chem. Int. Ed. Engl. 33, 2059, 1994; Carell et al., Angew. Chem. Int. Ed. Engl. 33, 2061; Gallop et al., J. Med. Chem. 37, 1233, 1994, *etc.*

The screening method of the present invention may be performed in various ways, and in particular, may be performed in a high throughput manner according to various binding assays, expression assays, or activity assay techniques known in the art.

The measurement of the changes in expression level of HMGCLL1 IS3 may be performed by various methods known in the art, for example, using RT-PCR (Sambrook et al., Molecular Cloning. A Laboratory Manual, 3rd ed. Cold Spring Harbor Press (2001)), northern blotting (Peter B. Kaufma et al., Molecular and Cellular Methods in Biology and Medicine, 102 to 108, CRC press), hybridization using cDNA microarray (Sambrook et al., Molecular Cloning. A Laboratory Manual, 3rd ed. Cold Spring Harbor Press (2001)), or *in situ* hybridization (Sambrook et al., Molecular Cloning. A Laboratory Manual, 3rd ed. Cold Spring Harbor Press (2001)).

In a case where the method is performed according to an RT-PCR protocol, first, the total RNA is isolated from the cells treated with a sample and the first strand cDNA is prepared using an oligo dT primer and reverse transcriptase. Then, PCR is performed using the first strand cDNA as a template along with an HMGCLL1 IS3-specific primer set. Examples of HMGCLL1 IS3-specific primer sets are shown in SEQ ID NOS: 12 and 13. Then, the PCR products are subjected to electrophoresis and the bands formed therefrom are analyzed to measure the changes in the expression level of HMGCLL1 IS3.

According to an embodiment of the present invention, the changes in the expression level of HMGCLL1 IS3 gene are determined by measuring the expression level of HMGCLL1 IS3.

The measurement of the expression level of HMGCLL1 IS3 may be performed by various analysis methods. For example, the expression level of HMGCLL1 IS3 may be measured by various immunoassay formats using the antibodies that specifically bind to HMGCLL1 IS3.

These immunoassays can be performed according to various quantitative immunoassay protocols developed previously. These immunoassay formats include radioimmunoassay, radioimmunoprecipitation, immunoprecipitation, enzyme-linked immunosorbent assay (ELISA), capture-ELISA, inhibition or inhibition or competition assay, sandwich immunoassay, immunofluorescent staining, and immunoaffinity purification, but the methods are not limited thereto. These immunoassays or immunostaining methods are described in Enzyme Immunoassay, E. T. Maggio, ed., CRC Press, Boca Raton, Florida, 1980; Gaastra, W., Enzyme-linked immunosorbent assay (ELISA), in Methods in Molecular Biology, Vol. 1, Walker, J.M. ed., Humana Press, NJ, 1984; and Ed Harlow and David Lane, Using Antibodies: A Laboratory Manual, Cold Spring Harbor Laboratory Press, 1999, and these are incorporated herein by reference.

For example, when the method of the present invention is performed according to the radioimmunoassay, antibodies labeled with radioisotopes (*e.g*., ¹⁴C, ¹²⁵I, ³²P, and ³⁵S) may be used for the detection of HMGCLL1 IS3.

When the method of the present invention is performed according to the ELISA method, in a specific embodiment of the present invention, the method includes (a) coating the surface of a solid substrate with an unknown cell sample lysate to be analyzed, (b) reacting the HMGCLL1 IS3-specific antibody as a primary antibody with the cell lysate, (c) reacting the resultant of step (b) with an enzyme-linked secondary antibody, and (d) measuring the activity of the enzyme.

Suitable as the solid substrate are hydrocarbon polymers (*e.g*., polystyrene and polypropylene), glass, metal, or gel, and most preferably microtiter plates.

The enzymes linked to the secondary antibody may include those which catalyze a chromogenic reaction, a fluorescent reaction, a luminescent reaction, or an infrared reaction, but the enzymes are not limited thereto, and include, for example, alkaline phosphatase, β-galactosidase, horseradish peroxidase, luciferase, and cytochrome P450. When alkaline phosphatase is used as the enzyme to be linked to the second antibody, chromogenic reaction substrates such as bromochloroindolyl phosphate (BCIP), nitro blue tetrazolium (NBT), naphthol-AS-Bl-phosphate, and enhanced chemifluorescence (ECF) may be used. When horseradish peroxidase is used as the enzyme to be linked to the second antibody, substrates such as chloronaphthol, aminoethylcarbazole, diaminobenzidine, D-luciferin, lucigenin (bis-*N*-methyl acridinium nitrate), resorufin benzyl ether, luminol, Amplex Red reagent (10-acetyl-3,7-dihydroxyphenoxazine), *p*-phenylenediamine-HCl and pyrocatechol (HYR), 3,3',5,5'-tetramethylbenzidine (TMB), 2,2'-azino-di[3-ethylbenzthiazoline sulfonate] (ABTS), o-phenylenediamine (OPD), or naphthol/pyronine, glucose oxidase, *t*-nitroblue tetrazolium (NBT), and *m*-phenazine methosulfate (PMS) may be used.

When the method of the present invention is performed according to the capture-ELISA method, in a specific embodiment of the present invention, the method includes (a) coating the surface of a solid substrate with an antibody to HMGCLL1 IS3 as a capturing antibody; (b) reacting the capturing antibody with a sample of cells; (c) reacting the resultant of step (b) with a detecting antibody to which a label generating a signal is linked and which specifically reacts to HMGCLL1 IS3; and (d) measuring the signal generated from the label.

The detection antibody has a label that generates a detectable signal. The label may contain chemical materials (*e.g.,* biotin), enzymes (*e.g.,* alkaline phosphatase, β-galactosidase, horseradish peroxidase, and cytochrome P450), radioactive materials (*e.g*., ¹⁴C, ¹²⁵I, ³²P, and ³⁵S), fluorescent materials (*e.g*., fluorescein), chromogenic materials, chemiluminescent materials, and fluorescence resonance energy transfer (FRET), but the label material is not limited thereto. Various labels and labeling methods are described in Ed Harlow and David Lane, Using Antibodies:A Laboratory Manual, Cold Spring Harbor Laboratory Press, 1999.

In the ELISA and capture-ELISA methods, the measurements of the final enzyme activity and signals may be performed according to various methods known in the art. The detection of signals enables a quantitative analysis of HMGCLL1 IS3. When biotin is used as a label, signals can easily be detected using streptavidin, whereas when luciferase is used as a label, signals can easily be detected using luciferin.

As a result of the measurement of the expression level of HMGCLL1 IS3, when the expression level of HMGCLL1 IS3 is measured to be down-regulated compared to that of the control group, the test material will be determined as a candidate material for preventing or treating CML and malignant tumors.

In still another aspect, the present invention provides a method for predicting prognosis of a malignant tumor.

Specifically, the method for predicting prognosis of a malignant tumor includes (a) measuring the expression level of HMGCLL1 IS3 from a biological sample isolated from a subject in which a malignant tumor has developed; and (b) comparing the measured expression level of HMGCLL1 IS3 with that of the control sample.

In the above method, when the expression level of HMGCLL1 IS3 measured in (a) is higher than that of a sample of the control group, the prognosis of the subject may be determined to be poor.

As described above, an increased expression level of HMGCLL1 IS3 is an indicator of an intrinsic or acquired resistance to currently available anticancer agent such as a kinase inhibitor , and thus, the prognosis of a disease after anticancer treatment may be expected to be poor in a patient in which HMGCLL1 IS3 is expressed at a higher level than that of the control group.

In still another aspect, the present invention provides a composition for predicting the prognosis of a malignant tumor which can be used for the method for predicting prognosis of a malignant tumor. Specifically, the composition may contain an agent capable of measuring the expression level of HMGCLL1 IS3.

The agent capable of measuring the expression level of HMGCLL1 IS3 is the same as described above.

In still another aspect, the present invention provides a kit for predicting the prognosis of a malignant tumor including the composition for predicting the prognosis of a malignant tumor.

In still another aspect, the present invention provides a method for determining drug resistance in a subject having a malignant tumor using a drug resistance-specific SNP.

Specifically, the method for determining drug resistance in a subject having a malignant tumor includes detecting or amplifying, from a biological sample isolated from a subject in which a malignant tumor has developed, a polynucleotide consisting of 2 to 51 continuous nucleotides, which include single nucleotide polymorphism (SNP) located at the 26^{th} nucleotide in a polynucleotide consisting of nucleotides selected from the group consisting of nucleotide sequences of SEQ ID NOS: 9 to 16 and 33 to 173; or a complementary polynucleotide thereof.

In the above method, when the polynucleotide is detected or amplified, the subject is determined to have drug resistance with respect to the malignant tumor treatment.

According to an embodiment of the present invention, the present inventors have preformed studies on SNPs which show statistical correlation between a group of patients having drug resistance to a CML targeting agent and a group of patients having no resistance to a CML targeting agent such as inatimib, nilotinib or dasatinib, etc.), and have attempted to predict or diagnose the drug tolerance of a CML patient based on the results of the study. As a result, the present inventors have identified that there is a correlation between particular SNPs in the HMGCLL1 gene and the expression level of HMGCLL1 IS3, and they have confirmed that the growth of CML cells and malignant tumor cells is effectively inhibited when the expression of the HMGCLL1 IS3 gene is inhibited.

Additionally, the genome-wide association analysis was performed for a total of 474 subjects (202 Korean patients and 272 Western patients) who were clinically diagnosed with CML (see Table 1). Cox's proportional hazard additive model was used as a statistical method. As a response index to drug response, the CML patients were administered with imatinib and then divided into a group with resistance to imatinib and a group without resistance to imatinib depending on whether each patient had reached the complete molecular response. As a result, it was confirmed that the gene which specifically appears in the imatinib-resistance group is the HMGCLL1 gene present on chromosome No. 6, and that this region is a novel gene region which has not been known to be associated with drug resistance. In particular, genotyping was performed for SNPs located in two candidate regions (chromosome Nos. 6 and 16), and as a result, the SNPs of rs10948926, rs10948927, rs9370435, rs4546489, rs4275061, rs9475323, rs9475327, and rs929679 present on chromosome No. 6 were finally identified, and these SNPs were confirmed to be associated with drug resistance of CML patients (see Table 3). Furthermore, a total of 141 SNPs having a homozygous haplotype with the same pattern as that of the 8 SNPs above were further identified, and this result suggests that the 141 SNPs are in high linkage disequilibrium (LD) relationship (r²>0.8) with the 8 SNPs and they are highly correlated with each other. This suggests that the 141 SNPs, which show the same haplotype pattern as that of the 8 SNPs, can also have an effect on the target gene, HMGCLL1.

In the method of the present invention, the polynucleotide may be obtained from various sources of a subject having a malignant tumor to be determined with respect to drug resistance, for example, from the blood of the subject.

In the method of the present invention, when the starting material is gDNA, the isolation of gDNA may be performed according to a conventional method known in the art (see Rogers & Bendich (1994)). When the starting material is an mRNA, the total RNA is isolated according to a conventional method known in the art (see Sambrook, J. et al., Molecular Cloning. A Laboratory Manual, 3rd ed. Cold Spring Harbor Press (2001); Ausubel, F.M. et al., Current Protocols in Molecular Biology, John Willey & Sons (1987); and Chomczynski, P. et al., Anal. Biochem. 162: 156 (1987)). The isolated total RNA is synthesized into cDNA using a reverse transcriptase. Since the total RNA is isolated from animal cells, it has a poly-A tail at the end of the mRNA and the cDNA can easily be synthesized using the oligo dT primers prepared using the characteristics of the sequence and a reverse transcriptase (see *PNAS* U.S.A., 85:8998 (1988); Libert F, et al., Science, 244:569 (1989); and Sambrook, J. et al., Molecular Cloning. A Laboratory Manual, 3rd ed. Cold Spring Harbor Press (2001)).

In the method of the present invention, the detection or amplification may be performed by applying various methods known in the art, which are used for confirming particular sequences. For example, the techniques that can be applied in the present invention may include fluorescent *in situ* hybridization (FISH), direct DNA sequence determination, pulsed-field gel electrophoresis (PFGE) analysis, southern blot analysis, single-strand conformation analysis (SSCA, Orita et al., PNAS, U.S.A. 86:2776 (1989)), RNase protection assay (Finkelstein et al., Genomics, 7:167 (1990)), dot-blot analysis, denaturing gradient gel electrophoresis (DGGE, Wartell et al., Nucl. Acids Res., 18: 2699 (1990)), a method using a mismatch recognition protein (*e.g.,* mutS protein of E. coli) (Modrich, Ann. Rev. Genet., 25: 229 to 253 (1991)), and allele-specific PCR, but the techniques are not limited thereto.

In still another aspect, the present invention provides a composition for determining drug resistance which can be used for determining drug resistance of a subject having a malignant tumor using a drug resistance-specific SNP.

Specifically, the composition may contain an agent capable of detecting or amplifying a polynucleotide consisting of 2 to 51 continuous nucleotides including single nucleotide polymorphism (SNP) located at the 26^{th} nucleotide in a polynucleotide consisting of nucleotides selected from the group consisting of nucleotide sequences of SEQ ID NOS: 9 to 16 and 33 to 173; or a complementary polynucleotide thereof.

The agent may be a primer or probe and the primer and probe are the same as described above.

In still another aspect, the present invention provides a kit for determining drug resistance which includes the composition for determining drug resistance.

In still another aspect, the present invention provides a method for determining the probability of developing a malignant tumor using a drug resistance-specific SNP.

Specifically, the method for determining the probability of developing a malignant tumor includes detecting or amplifying, from a biological sample isolated from a subject in which a malignant tumor has developed, a polynucleotide consisting of 2 to 51 continuous nucleotides including single nucleotide polymorphism (SNP) located at the 26^{th} nucleotide in a polynucleotide consisting of nucleotides selected from the group consisting of nucleotide sequences of SEQ ID NOS: 9 to 16 and 33 to 173; or a complementary polynucleotide thereof.

In the above method, when the polynucleotide is detected or amplified, the subject is determined to have a higher potential of developing a malignant tumor compared to the subject of the control group. For the purpose of determining the probability of developing a malignant tumor in a test subject, the control group can be a healthy individual.

In still another aspect, the present invention provides a method for predicting the prognosis of a malignant tumor using a drug resistance-specific SNP.

Specifically, the method for predicting the prognosis of a malignant tumor includes detecting or amplifying, from a biological sample isolated from a subject in which a malignant tumor has developed, a polynucleotide consisting of 2 to 51 continuous nucleotides including single nucleotide polymorphism (SNP) located at the 26^{th} nucleotide in a polynucleotide consisting of nucleotides selected from the group consisting of nucleotide sequences of SEQ ID NOS: 9 to 16 and 33 to 173; or a complementary polynucleotide thereof.

In the above method, when the polynucleotide is detected or amplified, it can be predicted that an anticancer treatment employing kinase inhibitors such as imanitib, nilotinib or dasatinib would not be effective.

In still another aspect, the present invention provides a composition for predicting the prognosis of a malignant tumor which can be used in a method for predicting the prognosis of a malignant tumor using a drug resistance-specific SNP.

Specifically, the composition may contain an agent capable of detecting or amplifying a polynucleotide consisting of 2 to 51 continuous nucleotides including single nucleotide polymorphism (SNP) located at the 26^{th} nucleotide in a polynucleotide consisting of nucleotides selected from the group consisting of nucleotide sequences of SEQ ID NOS: 9 to 16 and 33 to 173; or a complementary polynucleotide thereof.

The agent may be a primer or probe, and the primer and probe are the same as described above.

In still another aspect, the present invention provides a kit for predicting the prognosis of a malignant tumor, which contains the composition for predicting the prognosis of a malignant tumor.

### [DETAILED DESCRIPTION OF THE INVENTION]

Hereinafter, the present invention will be described in more detail with reference to the following Examples. However, these Examples are for illustrative purposes only and the invention is not limited by these Examples.

### EXAMPLES

### Example 1: Selection of groups of subjects

For the genome-wide association analysis, subjects were recruited with the approval of Research Center of Samsung Medical Center and Sungkyunkwan University. The groups of subjects consisted of patients clinically diagnosed of CML (*i.e.*, an experimental group of Korean patients (KOR discovery set) and a validation group of European patients (CEU validation set)) and the study was performed with prior approval.

The first group of subjects was an experimental group consisting of 202 Korean patients with CML, and these patients were divided into a group of 104 patients with CML who showed resistance and a group of 98 CML patients who showed no resistance, depending on whether they had reached the complete molecular response after taking imatinib as a response index to the drug. Upon examination of the missing frequency, it was discovered that the median value for the time when the complete molecular reaction appeared in each patient group of 201 patients, excluding one patient with high missing frequency, was 572.5 days in the patient group which showed resistance and 502 days in the patient group which showed no resistance, thus confirming that the complete molecular reaction appeared later in the patient group showing resistance.

The second group of subjects was the validation group consisting of 272 CML patients who were European-Canadians, and these patients were divided into a group of 122 CML patients who showed resistance, a group of 136 CML patients who showed no resistance, and a group of 14 CML patients who were not confirmed with respect to drug resistance, depending on whether they had reached the complete molecular response after taking imatinib, as a response index to the drug. A validation study was performed on different ethnic validation groups (see Table 1). Table 1 shows the number of subjects used in the experimental group and the validation group, and the groups were divided into a group responsive to CML and a group not responsive to CML.

**[Table 1] Number of subjects used in experimental group and validation group**

| | Response (1) | Non-response (0) | Missing (-9) | Total |
|---|---|---|---|---|
| KOR Discovery | 97 | 104 | 0 | 201 |
| CEU Validation | 135 | 121 | 14 | 270 |
| Total | 233 | 225 | 14 | 471 |

In the validation step, the median value for the time when the complete molecular reaction appeared in each patient group of 270 patients, excluding the samples of two patients with a high missing frequency, was 1,197 days in the patient group which showed resistance and 693 days in the patient group which showed no resistance, thus confirming that the complete molecular reaction appeared later in the patient group showing resistance (see Table 2).

**[Table 2] Time when CMR appeared in experimental group and validation group (Days)**

| | | Response (1) | Non-response (0) | Missing (-9) |
|---|---|---|---|---|
| KOR Discovery (N=201) | Mean | 602.05 | 739.25 | |
| | Median | 502 | 572.5 | |
| CEU Validation (N=270) | Mean | 1012.92 | 1373.33 | 813.08 |
| | Median | 693 | 1197 | 577 |

### Example 2: SNP Genotyping

Peripheral blood samples were collected from the subjects in each group during the course of treatment, and the purified double-stranded DNA genomic DNA product was determined using the QIAAMP® DNA Blood Maxi Kit (Qiagen Inc., Valencia, CA, U.S.A.) and was normalized to 50 ng/µL. For each sample, 5 µL of the normalized genomic DNA was used as a template for analysis of AFFYMETRIX® 6.0. With respect to the entire DNA samples of the experimental group, a total of 906,530 SNP genotypes were analyzed using the AFFYMETRIX® Genome-Wide Human SNP Array 6.0 (Affymetrix, Inc., Santa Clara, CA, U.S.A.). After genotyping, false genotype cluster patterns were excluded by visual inspection, and samples with missing genotype percentages of 5% or more were also excluded from the analysis. Additionally, 39,033 SNPs with missing genotype percentages of 5% or more and 198,053 SNPs with minor allele frequencies (MAFs) of 1% or less were sequentially excluded. As a result, a total of 637,886 autosomal SNPs were analyzed in the group of 201 patients.

In order to confirm the genotype analysis of the experimental group, a validation group was composed of the European cohort (a group of 272 patients) and a total of 25 SNP genotypes, excluding the 4 SNPs that failed in the primer design, were analyzed with respect to the 272 subjected in the validation group using the MASSARRAY® system (Sequenom, Inc., San Diego, CA, U.S.A.). In the validation group, the same quality control as in the experimental group was applied.

### Example 3: Statistical Analysis

The demographic structure of the samples was analyzed using multidimensional scaling (MDS) so as to confirm genetic homogeneity and to evaluate stratification. For multidimensional scaling (MDS), the AFFYMETRIX® 6.0 data for East Asians (Japanese (JPT) and Chinese (CHB)), Westerners (CEU), and Africans (YRI) was used in the International HapMap Project. The genomic inflation factor (λ) was calculated based on the median chi-squared statistic. The sensitivity between SNP markers and drug resistance was confirmed by measurement using Cox's proportional hazards model.

For the selection of gene locations to perform validation studies, candidate sites in which the P value is at least greater than 5.0×10⁻⁵ and at least 5 SNPs have a P value less than 0.0001 within 1 Mb were screened. For the corresponding sites, validation was performed in the European cohort. All of the statistical analyses including association analysis were performed using PLINK version 1.07 and R version 3.0.2. The structure of linkage disequilibrium (LD) was performed using HaploView version 4.1.10. Additionally, for the schematization of candidate sites, SNP Annotation and Proxy Search (SNAP) 2.2 were used.

Additionally, the present inventors performed SNP imputation so as to increase the analytical range of the genomic region for further analysis. The program IMPUTE2 version 2.3.0 was used to impute polymorphic SNPs that were not handled in the AFFYMETRIX® 6.0 array. Additionally, the haplotype estimation for SNP imputation was performed using SHAPEITv2.r727, and the reference panels used for SNP imputation were the 90 Japanese/Chinese (JPT + CHB) haplotypes known from the International HapMap Project data (Phase II Public Release # 22 NCBI Build 36) and 1000 genome project data (SHAPEIT2 in NCBI build 37 (hg19) coordinates).

### Example 4: Analysis results

### Example 4-1: Experimental group (Discovery set)

With respect to the group of subjects consisting of a group of 104 patients with CML who showed resistance and a group of 97 CML patients who showed no resistance depending on whether they had reached the complete molecular response after taking imatinib, among the group of subjects as an experimental group consisting of 201 CML patients, 637,886 SNP genotypes (MAF>1%) were evaluated. The multidimensional scaling (MDS) analysis showed that the genetic variants shown in Koreans are consistent with the variants according to the International HapMap Project data in the Japanese (JPT) and Chinese (CHB), but the genetic variants shown in Koreans were apparently distinct from those in Westerners (CEU) and Africans (YRI).

In the genome-wide association analysis with respect to Korean experimental group (discovery set, n=201), two genetic loci satisfied the significant criteria (minimum P value <5.0×10⁻⁵, P<0.0001 for at least 5 SNPs within 1 Mb). The two genetic loci are 6p12.1 and 16q23.3. For each genetic locus, a minimum P value of 2.25×10⁻⁵ to 4.64×10⁻⁶ was observed. Annotated genes, which are candidate genes potentially associated with drug resistance, such as HMGCLL1, GFRAL, BMP5, CDH13, and HSBP1, are located near the genetic loci.

### Example 4-2: Validation group (Validation set)

For the confirmation of genotype analysis of the experimental group, the validation group was composed of 272 patients of the European cohort, and the SNP genotypes of 272 subjects of the validation group were analyzed using the MASSARRAY® system (Sequenom, Inc., San Diego, CA, U.S.A.). Patients with a high proportion of missing genotypes were excluded from the analysis. Cox's proportional hazards model was performed for the validation group with a total of 270 subjects (121 in a group with resistance, 135 in a group with no resistance, and 14 in a group with deletion of phenotypic information) in the same manner as in the experimental group, and as a result, 8 SNPs (rs10948926, rs10948927, rs9370435, rs4546489, rs4275061, rs9475323, rs9475327, and rs9296791) showed significant association at the genetic locus 6p12.1.

The P values of the SNPs in the validation group ranged from 0.036 to 0.049, and the P values of the corresponding SNPs were within the range of 2.25×10⁻⁵ to 7.61×10⁻⁵. With reference to a hazard ratio, the association direction in the European cohort was the same as that of the experimental group, the Korean cohort. Referring to the rs9370435, which showed the most significant P value as the representative SNP, the frequency of the G allele in the experimental group was higher in the group with resistance (0.37 *vs.* 0.45), and the frequency of the G allele in the European validation group was also higher in the group with resistance (0.29 vs. 0.35), thus showing a similar tendency. The hazard ratio was 0.52 (95% confidence interval (CI), 0.38 to 0.71) in the Korean experimental group and 0.75 (95% confidence interval (CI), 0.57 to 0.98) in the European validation group. Table 3 shows frequencies and statistically significant hazard ratios of the 8 SNPs, which were confirmed through the validation group on chromosome No. 6 (6p12.1) of the present invention, in the experimental group and the validation group, respectively.

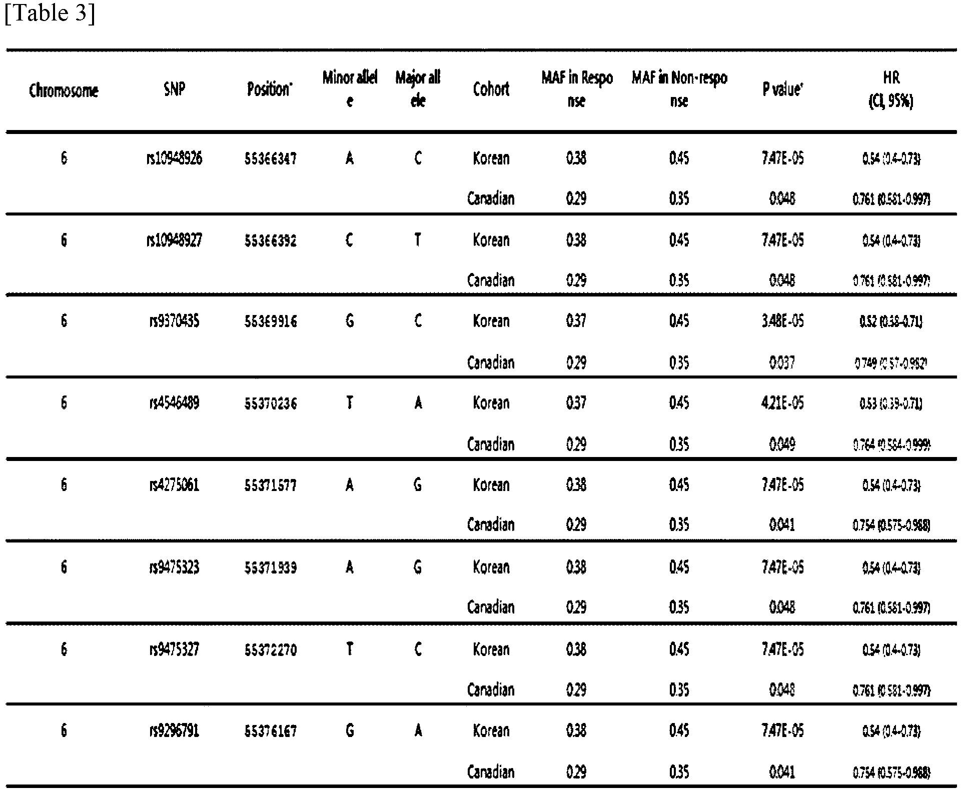

**[Table 4] Sequences of 8 SNPs**

| No | dbSNP | Genetic Loci | Sequences (5'-3') and SEQ ID NOS |
|---|---|---|---|
| 1 | rs10948926 | 6:55366347 | TCATGAATATGAACTGAATTCTCTT[A/C]TTTGACAA GCGGCCAAAGCCAGCTT (SEQ ID NO: 9) |
| 2 | rs10948927 | 6:55366392 | CAGCTTCTGGACTTCTGCTCTTCTC[C/T]AACAATCTA TTACAGTGGTTCTCAA (SEQ ID NO: 10) |
| 3 | rs9370435 | 6:55369916 | TAGTCTGTAAAAACATGATATAAAA[C/G]AAGACGT GAAGTGTTTCAGTCTTTA (SEQ ID NO: 11) |
| 4 | rs4546489 | 6:55370236 | TTGCTACATTCATGTGGAATACACC[A/T]TTCTGTAA GCAACACAAAATAACAT (SEQ ID NO: 12) |
| 5 | rs4275061 | 6:55371577 | ATAGTGAGGTTGGCAACTTGGATAC[A/G]CCAAAAA GAATCTGCAAAGTGCTTC (SEQ ID NO: 13) |
| 6 | rs9475323 | 6:55371939 | CTCCTGAAACTGGTGACCCCAAGCC[A/G]TTACTGCA GGTAAAGGACATTTCAC (SEQ ID NO: 14) |
| 7 | rs9475327 | 6:55372270 | TGAATATGTTCCCAATGATCTGGGC[C/T]GGACACTT CAGGTAATACTTCATGT (SEQ ID NO: 15) |
| 8 | rs9296791 | 6:55376167 | ACGTTATGTACAGTTCTGAGCGTGT[A/G]TTAAAAT GTGTACACATGCATGTG (SEQ ID NO: 16) |

### Example 5: Fine mapping

In the present invention, haplotypes were constituted with respect to the 8 SNPs (rs10948926, rs10948927, rs9370435, rs4546489, rs4275061, rs9475323, rs9475327, and rs9296791) associated with the drug resistance of CML patients. The 8 SNPs exhibited high linkage disequilibrium (LD) values, which indicate that the 8 SNPs are related to one another in a mutually dependent manner. These 8 SNPs are located at intron 5 (between exon 6 and exon 7) of the HMGCLL1 gene at chromosome 6p12.1.

To locate functional variants that are directly associated with the clinical imatinib response of CML patients, targeted sequencing was performed for a group of a total of 15 Korean patients (consisting of 8 patients in the response group which did not show resistance regarding the ACGTAATG and CTCAGGCA haplotypes as homozygous genotypes and 7 patients in the non-response group) and for a group of a total of 15 Westerner patients (consisting of 8 patients in the response group and 7 patients in the non-response group) under the same conditions as the Korean patients. That is, the target sequencing was performed for groups of a total of 30 Korean and Westerner patients. For the target sequencing, the Ion Torrent Personal GENOME MACHINE™ (PGM™) platform, a next-generation nucleotide sequence analysis method, was used, and Ampliseq, a PCR-based enrichment method, was used as the capture method.

Basic data analysis was performed using the Torrent Suite 4.0.2 and read mapping was performed using the TMAP in the hg19 reference genome. For variants, annotations were added to each of the variants via ANNOVAR following the performance through VariantCaller (v4.0-r76860).

From the results of Ampliseq, the present inventors could not identify coding variants (exonic variants) which are directly related to changes in protein sequences, but they did identify SNPs which are located around the exons and thus capable of affecting the transcription of genes. Additionally, the present inventors confirmed that the 8 SNPs located at the introns are located in a large LD block including exon 6 and exon 7, suggesting that these variants may affect the target gene (HMGCLL1).

**[Table 5] MassARRAY primer pair capable of amplifying SNPs of the present invention**

| SNP | Primer | Sequence (5' to 3') |
|---|---|---|
| rs10948927 | Forward | ACGTTGGATGAAAGCCAGCTTCTGGACTTC (SEQ ID NO: 17) |
| rs10948927 | Reverse | ACGTTGGATGAGCACCAAGGTGATTCTGTG (SEQ ID NO: 1 8) |
| rs9475327 | Forward | ACGTTGGATGGCCCTCAAACTAAACCCAAC (SEQ ID NO: 19) |
| rs9475327 | Reverse | ACGTTGGATGGGAACATGAAGTATTACCTG (SEQ ID NO: 20) |
| rs9475323 | Forward | ACGTTGGATGTGAGTCCAAACCTATTCCTG (SEQ ID NO: 21) |
| rs9475323 | Reverse | ACGTTGGATGGATCTCCTGAAACTGGTGAC (SEQ ID NO: 22) |
| rs4546489 | Forward | ACGTTGGATGCATAGGAGCTACTTTGCTAC (SEQ ID NO: 23) |
| rs4546489 | Reverse | ACGTTGGATGGGCATGTTATTTTGTGTTG (SEQ ID NO: 24) |
| rs10948926 | Forward | ACGTTGGATGAAGTCCAGAAGCTGGCTTTG (SEQ ID NO: 25) |
| rs10948926 | Reverse | ACGTTGGATGAGTGAGAAAAGGCCTCTACC (SEQ ID NO: 26) |
| rs4275061 | Forward | ACGTTGGATGTAATGATGGACCCAAAGCCC (SEQ ID NO: 27) |
| rs4275061 | Reverse | ACGTTGGATGCTTAAAGGAAGCACTTTGCAG (SEQ ID NO: 28) |
| rs9370435 | Forward | ACGTTGGATGGGTAAGGTTATTAAAGACTG (SEQ ID NO: 29) |
| rs9370435 | Reverse | ACGTTGGATGGTATTTGTGCTTGTTGAGGTC (SEQ ID NO: 30) |
| rs9296791 | Forward | ACGTTGGATGCCACACACTCACTGAAACAC (SEQ ID NO: 31) |
| rs9296791 | Reverse | ACGTTGGATGGTCCCAGACGTTATGTACAG (SEQ ID NO: 32) |

Additionally, a total of 141 SNPs (SEQ ID NOS: 33 to 173) having the same pattern of homozygous haplotypes as the 8 SNPs in Koreans and Westerners were further confirmed. These SNPs are highly associated with the 8 SNPs with a high linkage disequilibrium (LD) association (r²>0.8). That is, it is suggested that the 141 SNPs with the same haplotype pattern as the 8 SNPs may also affect the target gene (HMGCLL1).

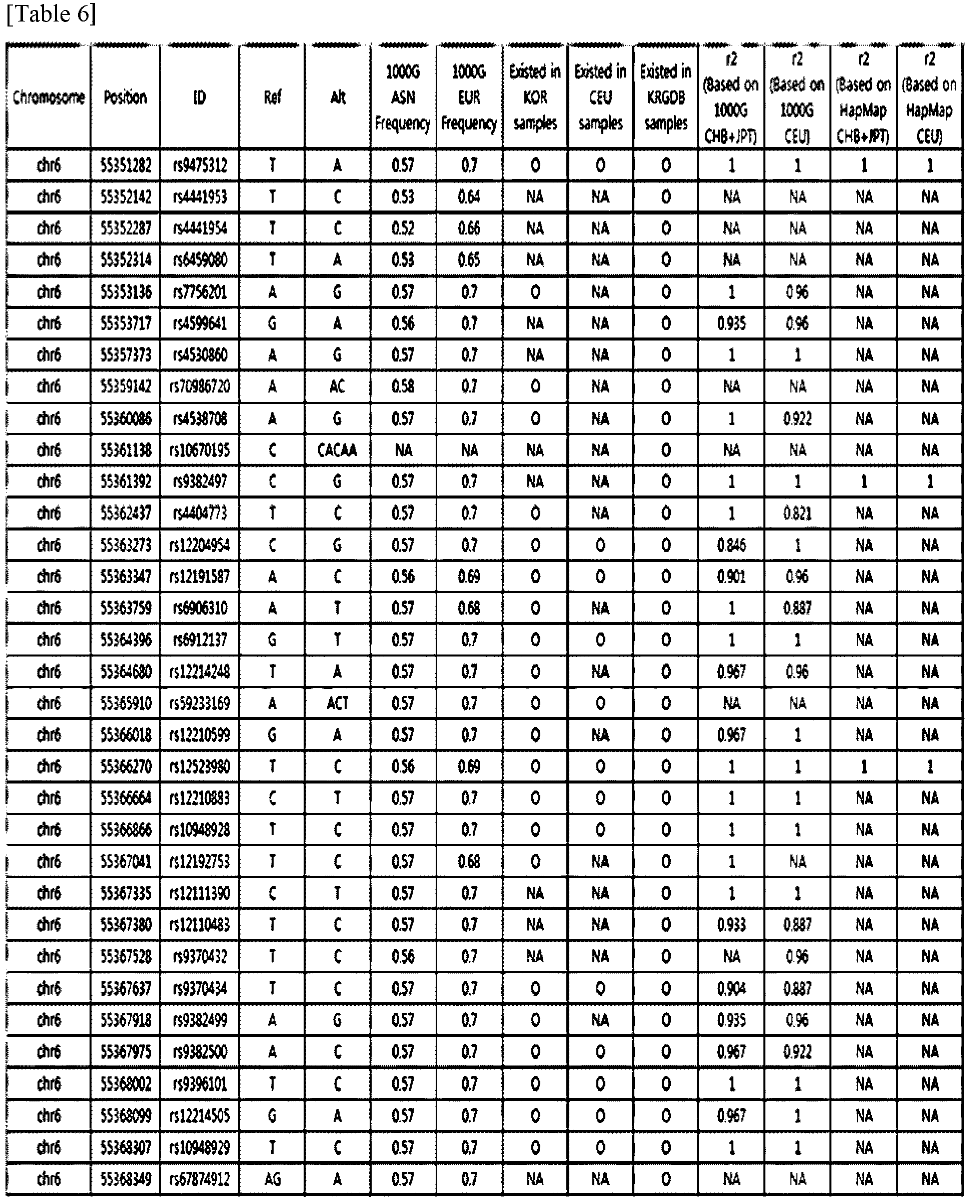

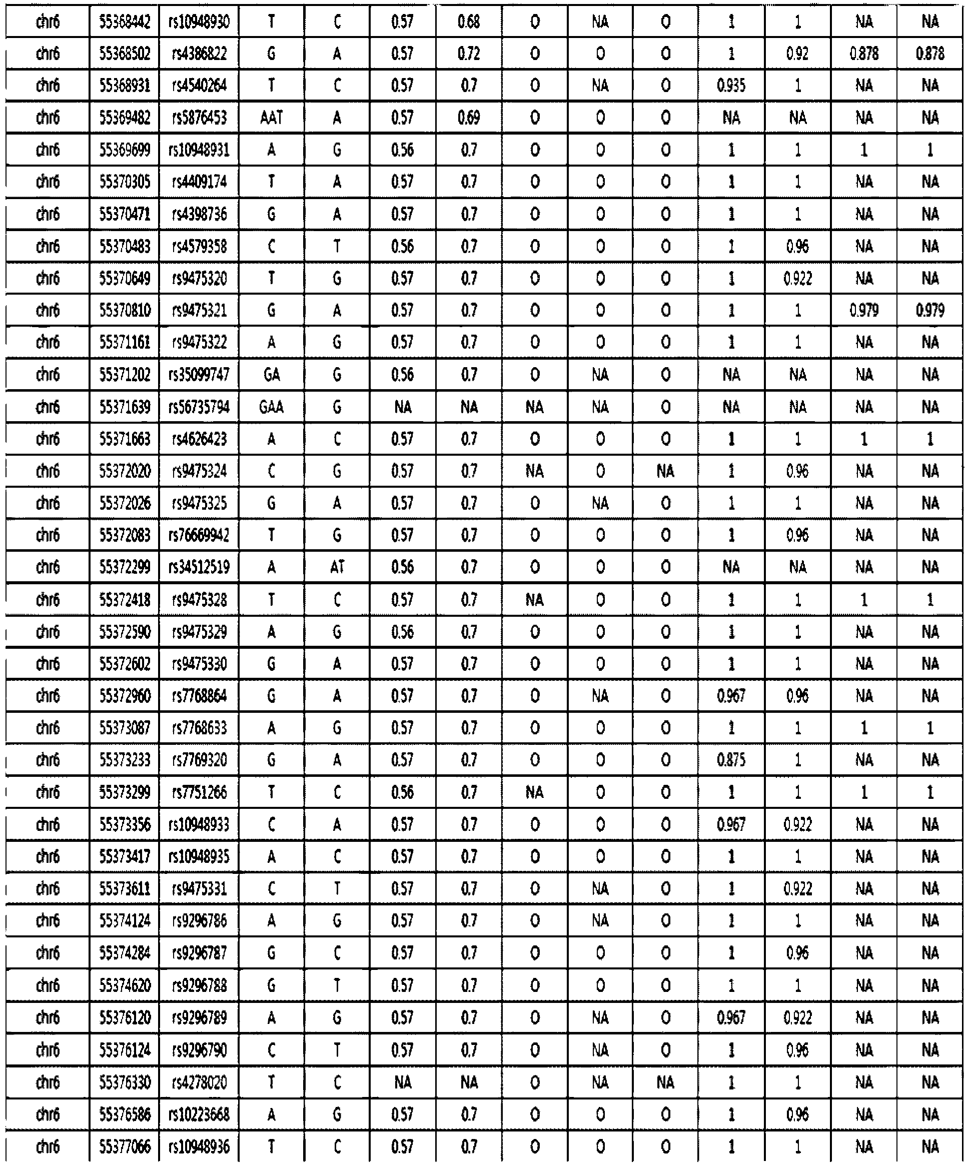

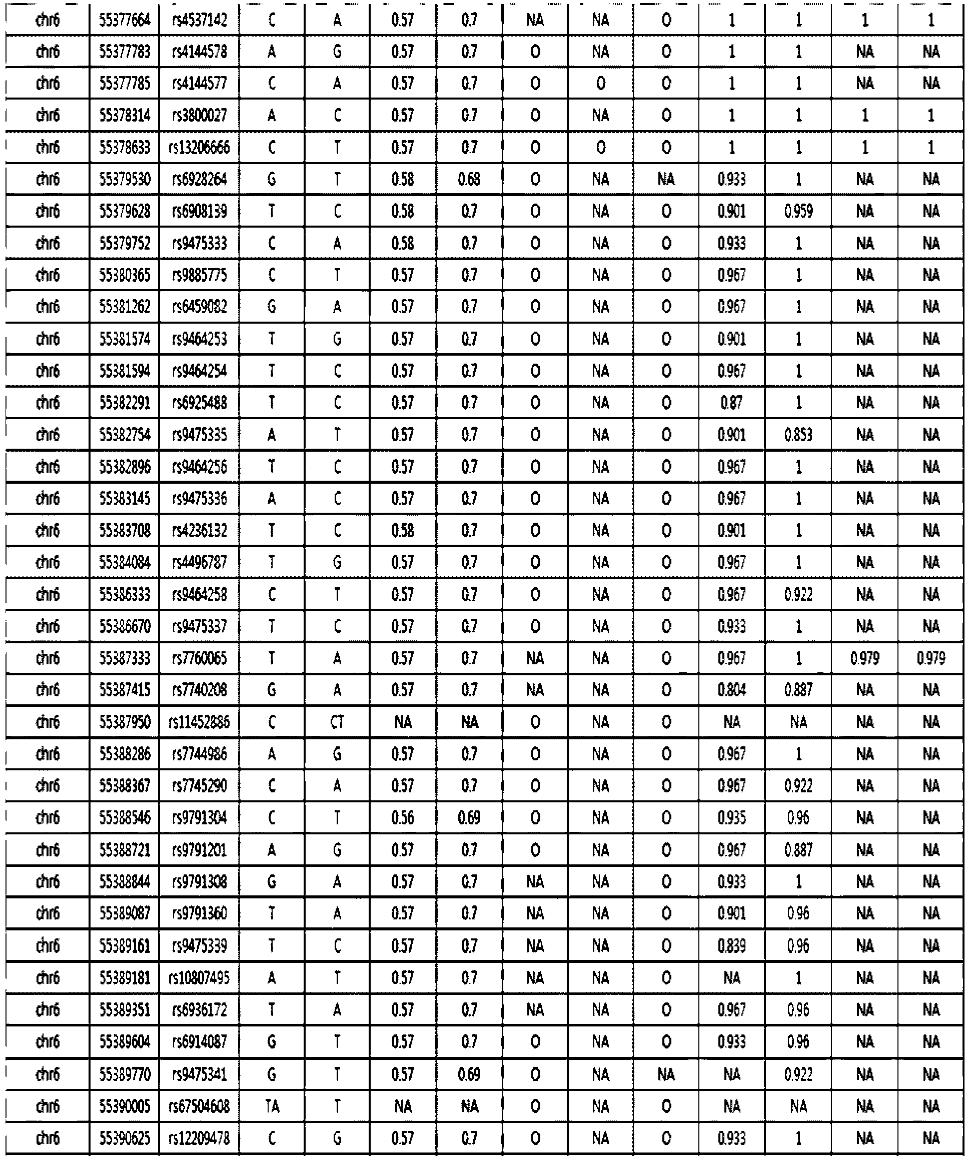

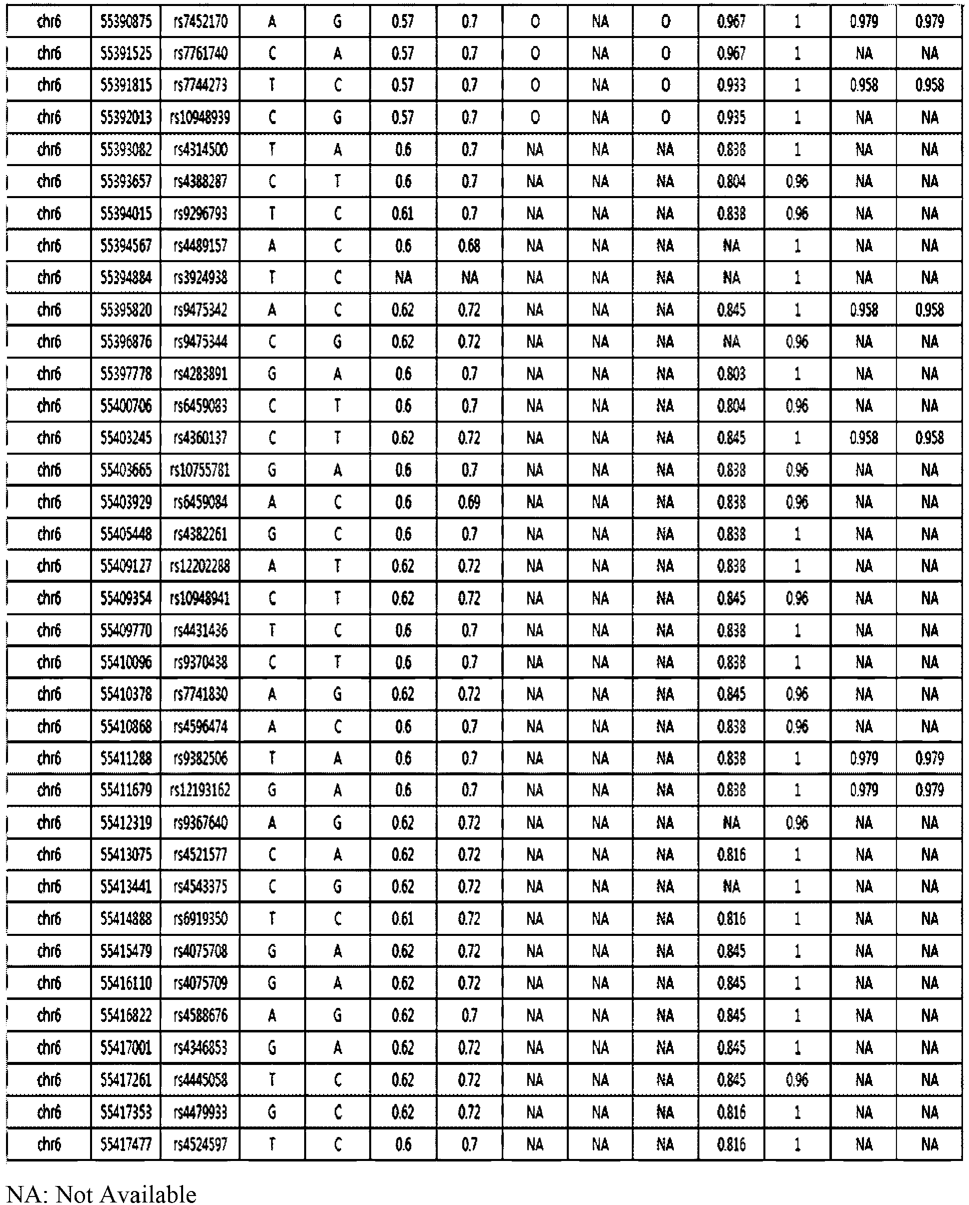

r² is confirmed by the association between rs10948926, which represents haplotypes, and the corresponding variants.

1000G and HapMap database are confirmed by SNP Annotation and Proxy Search (SNAP) through Proxy Search.

### Example 6: Cumulative incidence plot

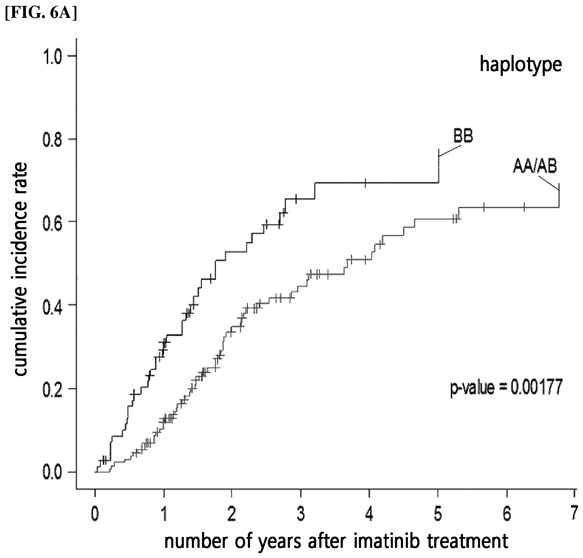

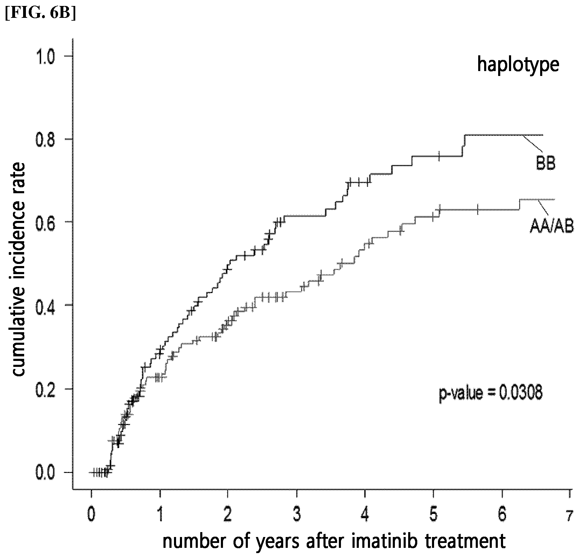

FIG. 6 graphically illustrates the cumulative incidence in Koreans and Westerners as a result of Example 4 analysis. The cumulative incidence plots correspond to graphs expressed in consideration of the time-point when a patient who had received an imatinib drug developed an MR4.5 event (complete molecular response) and discontinued the drug prescription. These graphs were expressed as follows using the R survival package: a case where patients have an ACGTAATG haplotype corresponding to a risk factor (AA/AB; AA is a group having a homogenous haplotype of ACGTAATG and AB is a group having an ACGTAATG haplotype and a CTCAGGCA haplotype); and a case where patients do not have an ACGTAATG haplotype corresponding to a risk factor (BB is a group having a homogenous haplotype of CTCAGGCA).

The P value of the cumulative incidence rate in Koreans was 0.00177 and the P value of the cumulative incidence rate in Westerners was 0.0308, thus showing a high level of significance. That is, these results show that the presence of the ACGTAATG haplotype confirmed by the present inventors is significantly associated with the drug response in a patient group.

### Example 7: Alternative splicing QPCR

In the present invention, for the confirmation of the effect of genetic polymorphism of SNPs on the expression of the subject gene (HMGCLL1), National Center for Biotechnology Information (NCBI), a joint scientific project between the European Bioinformatics Institute and the Wellcome Trust Sanger Institute (Ensembl), and Universal Protein Resource (UniProt) database were confirmed, and thereby at least 5 kinds of isoforms present in the gene were identified (Table 7).

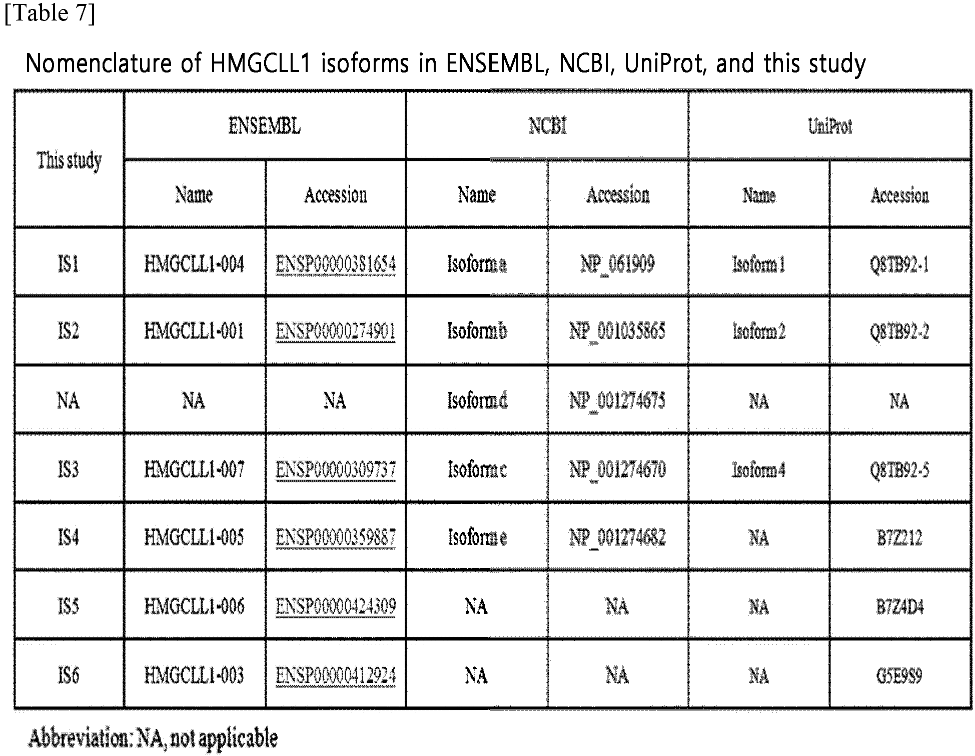

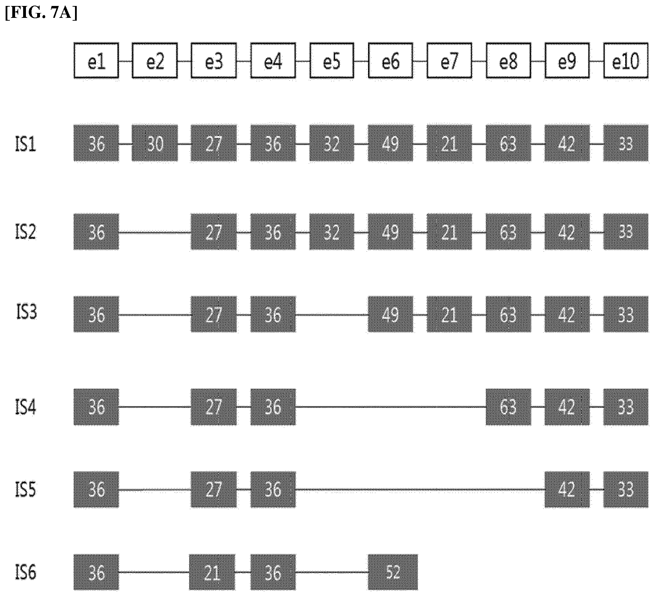

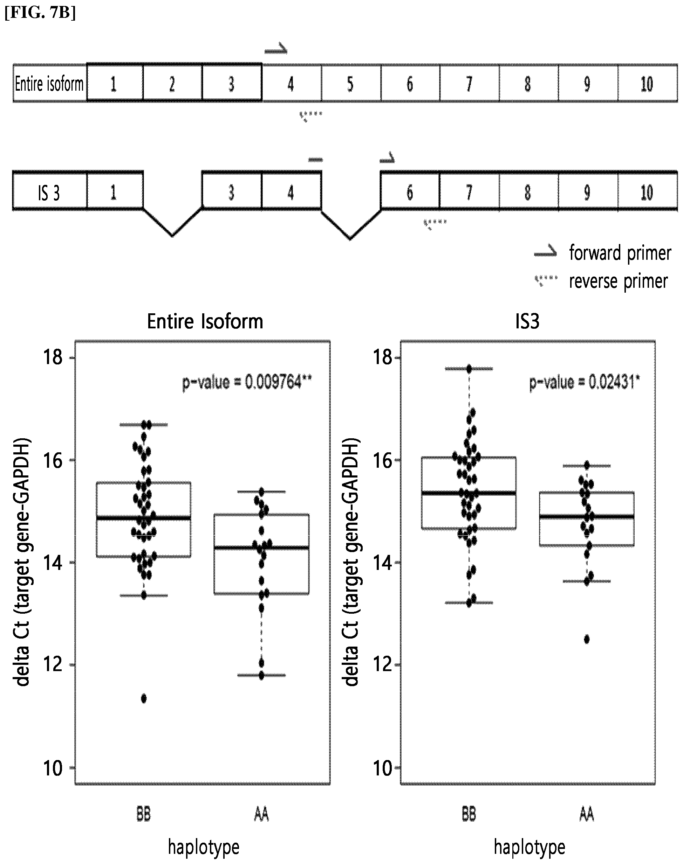

A quantitative polymerase chain reaction (QPCR) primer for quantifying the expression level of each of the identified isoforms was prepared (FIGS. 7A and 7B). The primer sequences for isoform 3 are as follows (see Table 8).

**[Table 8] QPCR primer pair capable of quantifying expression level of isoform 3**

| Target | Primer | Sequence (5' to 3') |
|---|---|---|
| Isoform 3 | Forward | TACCACAGGTTGCTGCTGGAG (SEQ ID NO: 174) |
| | Reverse | TGCAGACTTAACAACCTCCTCAA (SEQ ID NO: 175) |

Genotyping was performed in a total of 120 healthy normal people with respect to the 8 SNPs associated with drug resistance (rs10948926, rs10948927, rs9370435, rs4546489, rs4275061, rs9475323, rs9475327, and rs9296791) using the MASSARRAY® system (Sequenom, Inc., San Diego, CA, U.S.A.), and haplotypes were constituted based on the results. With respect to the distribution of each haplotype, three haplotypes (AA, AB, BB; AA is a group having a homogenous haplotype of ACGTAATG, AB is a group having an ACGTAATG haplotype and a CTCAGGCA haplotype, and BB is a group having a homogenous haplotype of CTCAGGCA) were shown to be distributed in a ratio of 0.37:0.48:0.15. As a result of confirming the expression levels quantified by QPCR for each isoform and statistical significance, isoform 3 showed the lowest P value. In particular, the comparison of homozygotes revealed that the P value was 0.04735 (Wilcoxon rank sum test), thus confirming that the expression level of the corresponding isoform was related to the haplotype. That is, it was confirmed that the distribution of haplotypes was significantly associated with the expression of particular isoforms, and this fact indicates that the genotype of the corresponding haplotype can affect selective splicing of the gene (HMGCLL1).

### Example 8: siRNA Transfection

In the present invention, *in vitro* experiments were performed using a CML cell line (K562) and siRNA (small interfering RNA) so as to confirm how important roles are being played in the cell by the isoforms confirmed through QPCR. The statistical association between the expression level of a gene according to each isoform quantified through QPCR and the genotype of the gene was confirmed. Specifically, as a result of the quantification of expression levels of isoforms by QPCR, the following facts were shown to be statistically significant.

When the difference in the total expression level of HMGCLL1 mRNA was confirmed by QPCR quantification in a sample group having a homogeneous genotype, the P value was 0.009764 in the group having a homogeneous haplotype of ACGTAATG (AA) and in the group not having the homogeneous haplotype of ACGTAATG (BB), thus showing a high level of significance. Additionally, as a result of selective confirmation in isoform 3, the P value was 0.02431 in the group having a homogeneous haplotype of ACGTAATG (AA) and in the group not having the homogeneous haplotype of ACGTAATG (BB), thus showing a significant result. The primer binding sites for quantifying the corresponding isoforms and the resulting quantified values are shown in FIG. 7B. Each graph was prepared using the program R.

### Example 9: Effect of HMGCLL1 IS3 siRNA on somatic mutation cell line of T315I

A cell line in which a somatic mutation of T315I was induced was prepared using the Clontech Guide-it CRISPR/Cas9 System and inoculated into a 96-well plate in which DMEM medium containing 10% FBS, 100 units/mL penicillin, and 100 µg/mL streptomycin at a concentration of 3×10³ cells/well and cultured under the conditions of 37°C and 5% CO₂.

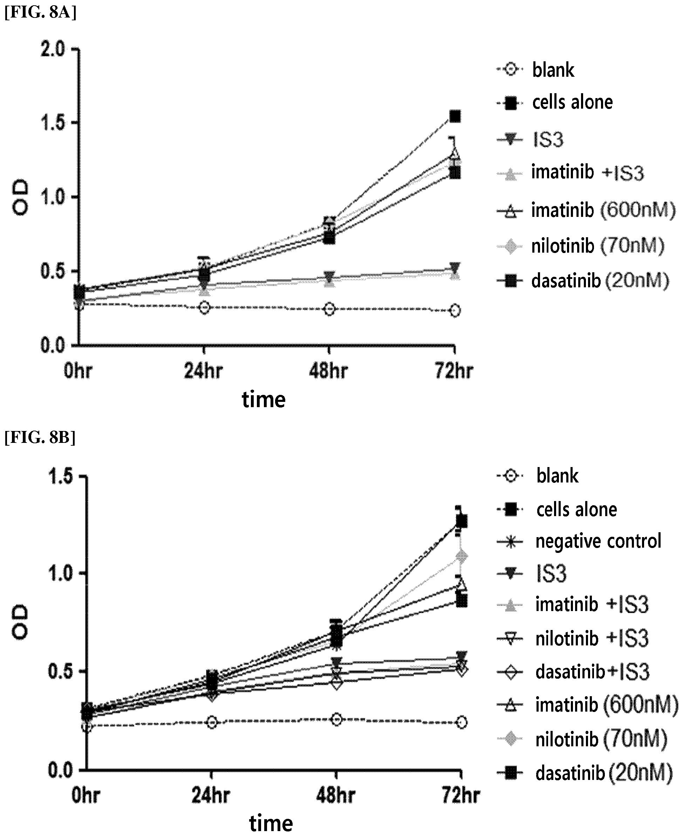

The cultured cell line was treated with each of HMGCLL1 IS3 siRNA, a first generation anticancer agent (imatinib) or a second generation anticancer agent (nilotinib or dasatinib), or a combination thereof, and the survival rates of the cell line were compared (FIGS. 8A and 8B). In particular, two kinds of siRNAs having the nucleotide sequences of SEQ ID NOS: 5 and 176 to 178 shown below (negative control siRNA or IS3 siRNA) were used as the siRNAs. The cell line was treated with imatinib, nilotinib, or dasatinib, each 10 nM, with IS3 siRNA, or IS3 siRNA alone, or imatinib (600 nM), nilotinib (70 nM), or dasatinib (20 nM), alone, and the survival rates of the cell line were confirmed using the Cell counting Kit-8 (CCK-8 assay) and water-soluble tetrazolium salt (WST-8).

**[Table 9] siRNA Sequence for knockdown of particular region of corresponding gene and sequence of corresponding C911 control group**

| Target | Category | siRNA Sequence (5' to 3') |
|---|---|---|
| Negative control, C911 | Sense | GGUACCACUCCUUGCUGCU (SEQ ID NO: 176) |
| | Antisense | AGCAGCAAGGAGUGGUACC (SEQ ID NO: 177) |
| HMGCLL1 Isoform 3 | Sense | GGUACCACAGGUUGCUGCU (SEQ ID NO: 178) |
| | Antisense | AGCAGCAACCUGUGGUACC (SEQ ID NO: 5) |

As shown in FIGS. 8A and 8B, it was confirmed that the first generation anticancer agent or the second generation anticancer agent alone did not affect the survival rate of the cell line in which the somatic mutation of T315I was induced.

In contrast, in a case where HMGCLL1 IS3 siRNA was used, both when the siRNA is used alone and when used in combination with the first generation anticancer agent or the second generation anticancer agent, the survival rate was reduced for the cell line in which the somatic mutation of T315I was induced. In particular, when the HMGCLL1 IS3 siRNA was used in combination with the first generation anticancer agent or the second generation anticancer agent, it was confirmed that the survival rate of the cell line was further significantly reduced.

### Example 10: Effect of HMGCLL1 IS3 siRNA on CML cell line

CML cell lines (K562 and CML-T1 cell line) and a normal lymphoblastoid cell line (LCL) were inoculated into a 96-well plate in which DMEM medium containing 10% FBS, 100 units/mL penicillin, and 100 µg/mL streptomycin at a concentration of 3×10³ cells/well and cultured under the conditions of 37°C and 5% CO₂.

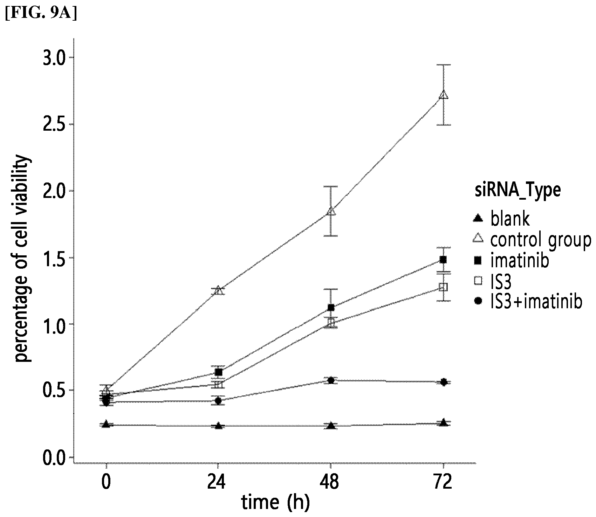

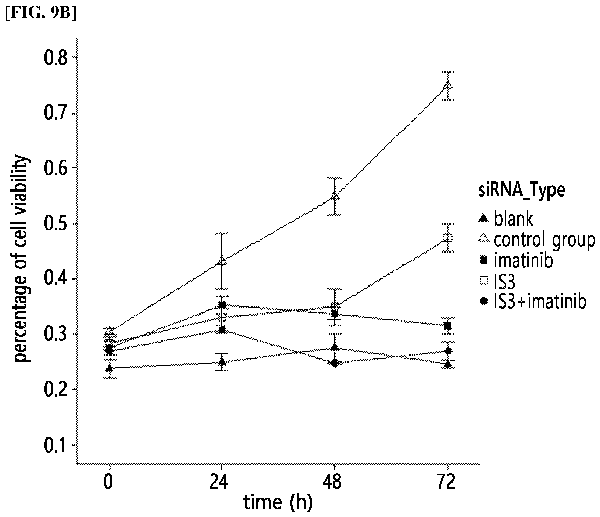

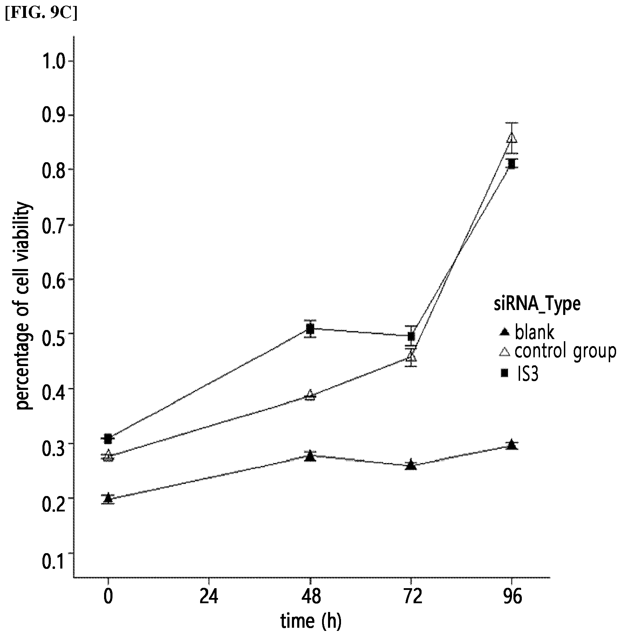

Each cultured cell line was treated with HMGCLL1 IS3 siRNA, and the first generation anticancer agent (imatinib), individually or in combination, and the survival rates of the cell lines were compared (FIGS. 9A to 9C). In particular, the siRNAs used had the nucleotide sequences of SEQ ID NOS: 5 and 176 to 178. The amount of imatinib was 0.6 µM per cell line and the survival rates of the cell lines were confirmed by the CCK-8 assay.

As shown in FIGS. 9A to 9C, the survival rates of the CML cell lines (K562 and CML-T1 cell line) were reduced by HMGCLL1 IS3 siRNA and imatinib; however, the survival rate of the normal LCL was not affected by HMGCLL1 IS3 siRNA.

### Example 11: Analysis of action effect of HMGCLL1 IS3 siRNA

In order to confirm the anticancer effect of HMGCLL1 IS3 siRNA, whose anticancer activity was confirmed in Example 10, on the CML cell lines through Phospho-CRKL analysis and cell cycle analysis.

### Example 11-1: Phospho-CRKL analysis

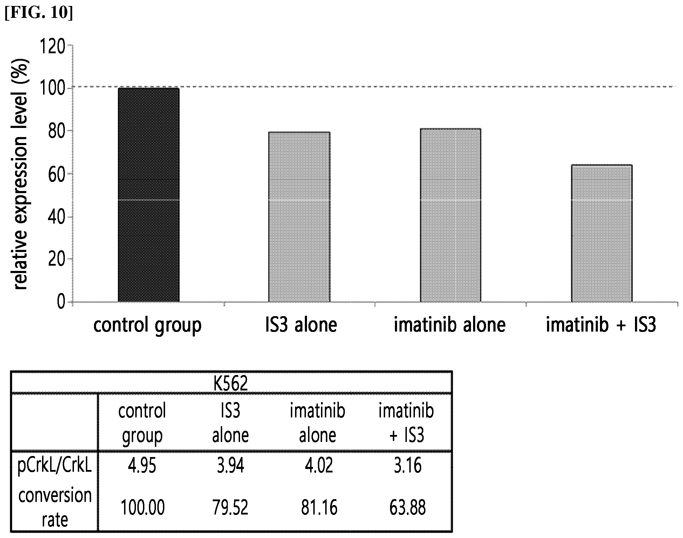

K562, a CML cell line, was treated with HMGCLL1 IS3 siRNA and the first generation anticancer agent (imatinib), individually or in combination. A ratio of phosphorylated CRKL and CRKL (phospho CrkL/CrkL ratio) of the treated cell line was calculated using the Phospho-CrkL (Tyr207) Colorimetric Cell-Based enzyme linked immunosorbent assay (ELISA) kit and the results were compared (FIG. 10).

As shown in FIG. 10, when K562 was treated with HMGCLL1 IS3 siRNA or the first generation anticancer agent (imatinib), the phospho CrkL/CrkL ratio was reduced to similar levels, whereas when K562 was treated with HMGCLL1 IS3 siRNA and the first generation anticancer agent (imatinib) in combination, the phospho CrkL/CrkL ratio was reduced to the lowest level, thus confirming the presence of a synergistic effect of the combination of HMGCLL1 IS3 siRNA and the existing anticancer agent.

### Example 11-2: Cell cycle analysis

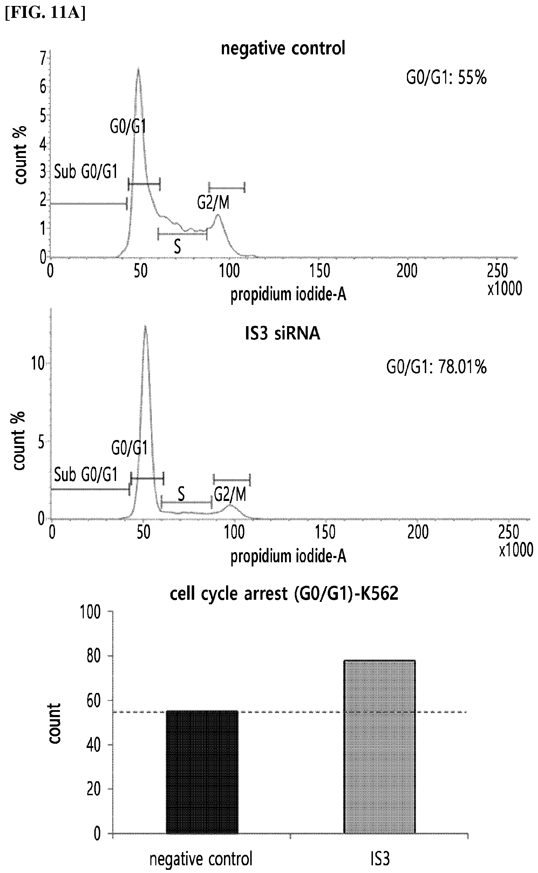

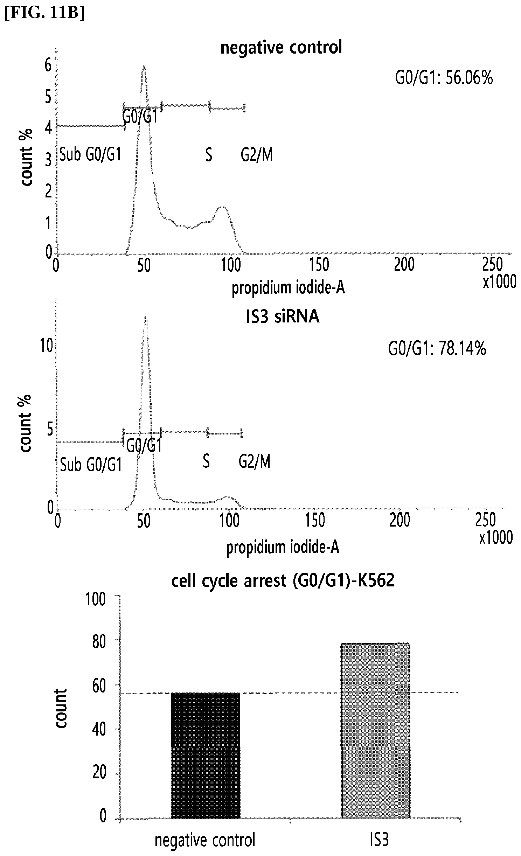

CML cell lines (K562 cells and LAMA-84 cells) were treated with the two kinds of siRNAs having the nucleotide sequences of SEQ ID NOS: 5 and 176 to 178 (negative control siRNA or IS3 siRNA) and the cells were immobilized. These cells were subjected to PI staining and applied to FACSVerse (BD Biosciences, Franklin Lakes, NJ, U.S.A.) so as to measure the percentage of the cells located in the G0/G1 phase (FIGS. 11A and 11B).

As shown in FIGS. 11A and 11B, it was confirmed that the percentage of the cells located in the G0/G1 phase was increased when CML cell lines (both K562 cells and LAMA-84 cells) were treated with IS3 siRNA.

As such, it was analyzed that the increase in the percentage of the cells located in the G0/G1 phase means that the cell cycle was arrested.

From the foregoing, it was confirmed that HMGCLL1 IS3 siRNA can inhibit signal transduction and arrest cell cycles by BCR-ABL1 by inhibiting the phosphorylation of CRKL in CML cell lines, thereby exhibiting anticancer activity.

### Example 12: Analysis of cancer activity of HMGCLL1 IS3 siRNA on various cancer cells

HMGCLL1 IS3 siRNA's anticancer activity with respect to representative cancer cell lines such as hematopoietic tissue or lymphatic tissue, autonomic ganglia, biliary tract, breasts, central nervous system, livers, pancreas, skin, stomach, urinary system, *etc.,* was determined.

Specifically, the cancer cell lines of hematopoietic tissue or lymphatic tissue (e.g., LAMA84 (chronic myeloid leukemia in blast crisis), KG-1 (acute myeloid leukemia, FAB M2), HL-60 (acute myeloid leukemia, FAB M2), NB-4 (acute promyelocytic leukemia), MOLM13 (acute myeloid leukemia, FAB M5B), CCRF-CEM (T acute lymphoblastic leukemia), and U-937 (histiocytic lymphoma)); solid cancer cell lines (*e.g.,* IMR-32 (neuroblastoma, KCLB, 10127), SNU-478 (ampulla of vater adenocarcinoma), MG-63 (osteosarcoma), Hs578T (breast invasive ductal carcinoma, KCLB, 30126), U-87MG (glioblastoma), SNU-886 (hepatocellular carcinoma), MIA-PaCa-2 (pancreatic ductal adenocarcinoma), SK-MEL-2 (malignant melanoma), RD (embryonal rhabdomyosarcoma), Hs 746T (gastric adenocarcinoma), and J-82 (bladder carcinoma)) were dispensed into a 96-well plate at a concentration of 3,000 to 5,000 cell lines/well and incubated in a wet incubator (37°C and 5% CO₂) having an environment similar to that of the human body and cultured for 3 days.

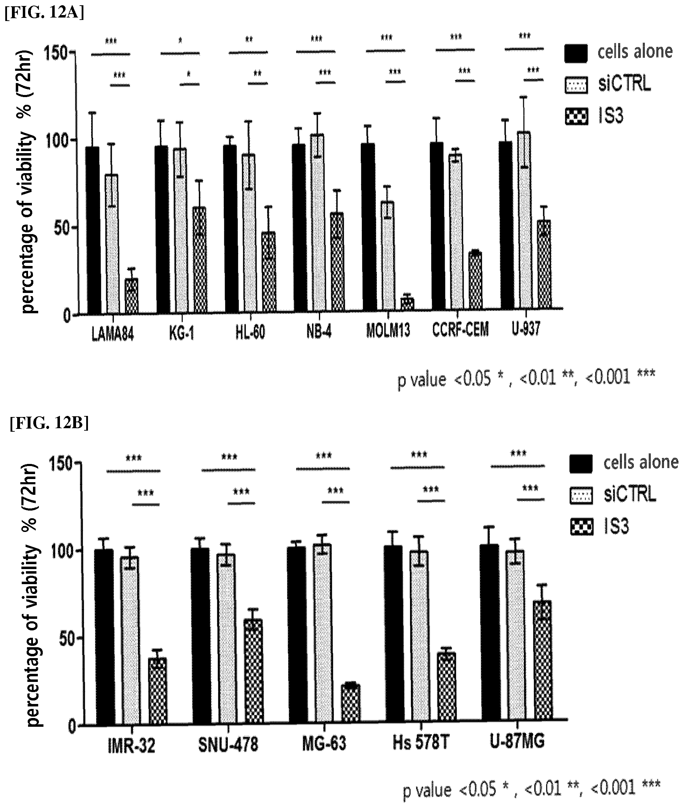

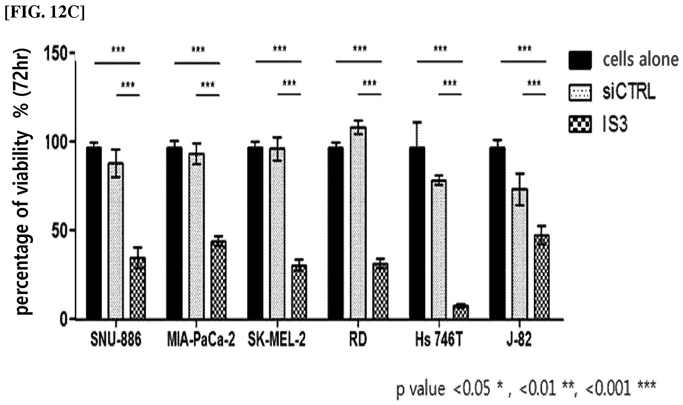

Each of the cultured cells was treated with the two kinds of siRNAs having the nucleotide sequences of SEQ ID NOS: 5 and 176 to 178 (negative control siRNA (siCTRL) or IS3 siRNA) at a concentration of 100 pmol based on 200,000 cells, cultured for up to 72 hours, and the survival rates of these cells were compared by the CCK-8 assay (FIGS. 12A to 12C).

As shown in FIGS. 12A to 12C, it was confirmed that IS3 siRNA significantly reduced the survival rate of each cell line in all of the cancer cell lines of hematopoietic tissue or lymphatic tissue and solid cancer cell lines.

While preferred embodiments of the present invention have been described in detail, it is obvious to one of ordinary skill in the art that these preferred embodiments are provided only for illustrative purposes and the scope of the present invention should not be limited thereto. Accordingly, the actual scope of the present invention will be defined by the appended claims and their equivalents.

## Claims

1. A pharmaceutical composition for the prevention or treatment of a malignant tumor, comprising an agent capable of inhibiting the expression or activity of 3-hydroxymethyl-3-methylglutaryl-CoA lyase like 1 isoform 3 (HMGCLL1 IS3).

2. The pharmaceutical composition of claim 1, wherein the agent is a polynucleotide which specifically binds to mRNA of HMGCLL1 IS3 and thereby inhibits translation of the same.

3. The pharmaceutical composition of claim 2, wherein the polynucleotide is an antisense oligonucleotide or siRNA.

4. The pharmaceutical composition of claim 2, wherein the polynucleotide specifically binds to part or the entirety of the nucleic acid region of the 190^{th} to the 446^{th} nucleotides of the nucleotide sequence of SEQ ID NO: 2, which encodes HMGCLL1 IS3 in the mRNA of HMGCLL1 IS3.

5. The pharmaceutical composition of claim 2, wherein the polynucleotide specifically binds to a part or entirety of the nucleotide sequence of SEQ ID NO: 4.

6. The pharmaceutical composition of claim 2, wherein the polynucleotide comprises any one nucleotide sequence selected from the group consisting of nucleotide sequences of SEQ ID NOS: 5 to 8.

7. The pharmaceutical composition of claim 2, wherein the polynucleotide comprises 19 to 40 continuous nucleotides, in which the polynucleotide comprises any one nucleotide sequence selected from the group consisting of nucleotide sequences of SEQ ID NOS: 5 to 8 and the polynucleotide is able to specifically bind to a part or entirety of the nucleotide sequence of SEQ ID NO: 4.

8. The pharmaceutical composition of claim 1, wherein the agent is selected from the group consisting of an aptamer, an antibody, a peptide, a fragment of the antibody, a low molecular weight compound, and a combination thereof, wherein the agent specifically binds to HMGCLL1 IS3 protein and thereby inhibits an activity of HMGCLL1 IS3 protein.

9. The pharmaceutical composition of claim 1, wherein the malignant tumor is hematologic malignancy.

10. The pharmaceutical composition of claim 1, wherein the malignant tumor is lymphoid malignancy.

11. The pharmaceutical composition of claim 1, wherein the malignant tumor is a solid cancer.

12. The pharmaceutical composition of claim 11, wherein the solid cancer is selected from the group consisting of neuroblastoma, adenocarcinoma, osteosarcoma, breast invasive ductal carcinoma, glioblastoma, hepatocellular carcinoma, pancreatic ductal adenocarcinoma, malignant melanoma, embryonal rhabdomyosarcoma, gastric adenocarcinoma, bladder carcinoma, and a combination thereof.

13. A pharmaceutical composition for the prevention or treatment of malignant tumors showing resistance to tyrosine kinase inhibitors, comprising an agent inhibiting an expression or activity of 3-hydroxymethyl-3-methylglutaryl-CoA lyase like 1 isoform 3 (HMGCLL1 IS3).

14. A method for determining drug resistance in a subject having a malignant tumor, comprising:
(a) measuring an expression level of HMGCLL1 IS3 from a biological sample of a test subject who has a malignant tumor; and
(b) comparing the measured expression level of HMGCLL1 IS3 of step (a) with that of a control sample.

15. The method of claim 14, wherein when the expression level of HMGCLL1 IS3 measured in (a) is higher than that of a sample of the control group, the subject is determined to have drug resistance to the malignant tumor.

16. A composition for diagnosing drug resistance comprising an agent capable of measuring the expression level of HMGCLL1 IS3.

17. The composition of claim 16, wherein the agent is an agent for measuring the level of the mRNA or protein of HMGCLL1 IS3.

18. The composition of claim 16, wherein the agent for measuring the mRNA level is a primer or probe.

19. The composition of claim 16, wherein the agent for measuring the protein level is selected from the group consisting of an aptamer, an antibody, a peptide, a fragment of the antibody, a low molecular weight compound, and a combination thereof, which specifically binds to the protein.

20. A kit for diagnosing drug resistance comprising the composition of any one of claims 16 to 19.

21. A method for determining the probability of developing a malignant tumor in a test subject, comprising:
(a) measuring an expression level of HMGCLL1 IS3 from a biological sample of a test subject who is suspected of the development of a malignant tumor; and
(b) comparing the measured expression level of HMGCLL1 IS3 of step (a) with that of a control sample.

22. The method of claim 21, wherein when the expression level of HMGCLL1 IS3 measured in (a) is higher than that of a sample isolated from the control subject, the subject is determined to have a higher probability of developing a malignant tumor than the control subject.

23. A method for screening a malignant tumor therapeutic agent, comprising:
(a) treating an isolated malignant tumor sample, in which HMGCLL1 IS3 is expressed, with a candidate material as a malignant tumor therapeutic agent; and
(b) comparing an expression level of HMGCLL1 IS3 measured for the sample treated in (a) with that of HMGCLL1 IS3 measured in a control sample which is the same isolated malignant tumor sample, but not treated with the candidate material.

24. The method of claim 23, wherein when the expression level of HMGCLL1 IS3 treated in (a) is lower compared to that measured in the control sample, the candidate material is determined to have a therapeutic effect for treating the malignant tumor.

25. A method for predicting prognosis of a malignant tumor, comprising:
(a) measuring an expression level of HMGCLL1 IS3 of a biological sample of a test subject who has a malignant tumor; and
(b) comparing the measured expression level of HMGCLL1 IS3 of (a) with that of a control sample.

26. The method of claim 25, wherein when the expression level of HMGCLL1 IS3 measured in (a) is higher than that of the control sample, the prognosis of the subject is determined to be poor.

27. A composition for predicting the prognosis of a malignant tumor comprising an agent capable of measuring the expression level of HMGCLL1 IS3.

28. The composition of claim 27, wherein the agent is an agent for measuring the level of the mRNA or protein of HMGCLL1 IS3.

29. The composition of claim 28, wherein the agent for measuring the level of the mRNA is a primer or probe.

30. The composition of claim 28, wherein the agent for measuring the level of the protein is selected from the group consisting of an aptamer, an antibody, a peptide, a fragment of the antibody, a low molecular weight compound, and a combination thereof, which specifically binds to the protein.

31. A kit for predicting the prognosis of a malignant tumor comprising the composition of any one of claims 27 to 30.

32. A method for determining drug resistance in a subject having a malignant tumor, which comprises detecting or amplifying, from a biological sample isolated from a subject in which a malignant tumor has developed, a polynucleotide consisting of 2 to 51 continuous nucleotides comprising single nucleotide polymorphism (SNP) located at the 26^{th} nucleotide in a polynucleotide consisting of nucleotides selected from the group consisting of nucleotide sequences of SEQ ID NOS: 9 to 16 and 33 to 173; or a complementary polynucleotide thereof.

33. The method of claim 32, wherein when the polynucleotide is detected or amplified, the subject is determined to have drug resistance to a malignant tumor.

34. A composition for diagnosing drug resistance, which comprises an agent capable of detecting or amplifying a polynucleotide consisting of 2 to 51 continuous nucleotides comprising single nucleotide polymorphism (SNP) located at the 26^{th} nucleotide in a polynucleotide consisting of nucleotides selected from the group consisting of nucleotide sequences of SEQ ID NOS: 9 to 16 and 33 to 173; or a complementary polynucleotide thereof.

35. The composition of claim 34, wherein the agent is a primer or probe.

36. A kit for diagnosing drug resistance comprising the composition of claim 34 or 35.

37. A method for determining the probability of developing a malignant tumor, which comprises detecting or amplifying, from a biological sample isolated from a subject in which a malignant tumor has developed, a polynucleotide consisting of 2 to 51 continuous nucleotides comprising single nucleotide polymorphism (SNP) located at the 26^{th} nucleotide in a polynucleotide consisting of nucleotides selected from the group consisting of nucleotide sequences of SEQ ID NOS: 9 to 16 and 33 to 173; or a complementary polynucleotide thereof.

38. The method of claim 37, wherein when the polynucleotide is detected or amplified, the subject is determined to have a higher probability of developing a malignant tumor than the control subject.

39. A method for predicting the prognosis of a malignant tumor, which comprises detecting or amplifying, from a biological sample isolated from a subject in which a malignant tumor has developed, a polynucleotide consisting of 2 to 51 continuous nucleotides comprising single nucleotide polymorphism (SNP) located at the 26^{th} nucleotide in a polynucleotide consisting of nucleotides selected from the group consisting of nucleotide sequences of SEQ ID NOS: 9 to 16 and 33 to 173; or a complementary polynucleotide thereof.

40. The method of claim 39, wherein when the polynucleotide is detected or amplified, the prognosis of the subject is determined to be poor.

41. A composition for predicting the prognosis of a malignant tumor, which comprises an agent capable of detecting or amplifying a polynucleotide consisting of 2 to 51 continuous nucleotides comprising single nucleotide polymorphism (SNP) located at the 26^{th} nucleotide in a polynucleotide consisting of nucleotides selected from the group consisting of nucleotide sequences of SEQ ID NOS: 9 to 16 and 33 to 173; or a complementary polynucleotide thereof.

42. The composition of claim 41, wherein the agent is a primer or probe.

43. A kit for predicting the prognosis of a malignant tumor comprising the composition of claim 41 or 42.
